# EUROPÄISCHE PATENTANMELDUNG

(11) **EP 4 684 871 A1**
(43) Veröffentlichungstag der Anmeldung: **28.01.2026**
(21) Anmeldenummer: 24191098.3
(22) Anmeldetag: 26.07.2024
(51) Int. Cl.: B01J 8/02, C07C 209/68

(54) **VERFAHREN ZUR KONTINUIERLICHEN, KATALYTISCHEN HYDRIERUNG VON MDA**

(71) Anmelder: Evonik Operations GmbH, 45128 Essen (DE)
(72) Erfinder: KUHLMANN, Hanns, 45549 Sprockhövel (DE); RIX, Armin Matthias, 45770 Marl (DE); PAUL, Niklas, 45770 Marl (DE); WESSNER, Lea, 44139 Dortmund (DE); VARGAZ GOMEZ, Maria, 45721 Haltem am See (DE); WINKLER, Tobias, 48249 Dülmen (DE); BOECK, Florian, 58455 Witten (DE); SUDHOFF, Daniel, 59192 Bergkamen (DE)
(74) Vertreter: Evonik Patent Association

(57) **Zusammenfassung**

Anlage zur Hydrierung von Methylendianilin (MDA; Edukt1) mit einem Wasserstoffgeber (Edukt2), insb. einem gasförmigen Wasserstoffgeber, vorzugsweises Wasserstoff (H2), umfassend eine Konditionierungseinheit für die Edukte, eine Reaktoreinheit und eine Trenneinheit, wobei
- die Konditionierungseinheit mind. eine Teillänge) der (Zu-)Leitungen für Edukte1, Edukt2 und mind. ein Lösungsmittel, mind. einen Wärmetauscher in mind. einer (Zu-)Leitung, mind. einen Mischer zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine (erste) Hauptreaktor
- einen ersten Strömungsweg für das Stoffgemisch
über die immobile Katalysatorpackung und
- einen weiteren hiervon getrennten, geschlossenen Strömungsweg für ein

Wärmetauschmittel außerhalb der Katalysatorpackung umfasst, und
wobei in den Medienumlauf ein Wärmetauscher eingebunden ist;
- die Trenneinheit mind.
- eine erste Trennstufe zur Abtrennung des Lösungsmittels und
- eine zweite Trennstufe zur Abtrennung vom Edukt und Nebenprodukten vom Produkt umfasst, wobei
i) ein Sammelkreislauf für einen geschlossenen ersten Medienumlauf umfasst ist, in den als mind. eine Wärmequelle der Wärmetauscher des Hauptreaktors und/oder ein Wärmetauscher der Trenneinheit sowie ein erster Verdampfer eingebunden sind,
ii) ein Zwischenkreislauf für einen geschlossenen zweiten Medienumlauf umfasst ist, in den der erste Verdampfer, mind. ein Verdichter und ein zweiter Verdampfer eingebunden sind, und wobei
iii) mind. ein Verteilerkreislauf für einen geschlossenen dritten Medienumlauf umfasst ist, in den als Wärmesenke mind. ein Wärmetauscher der Reaktoreinheit , mind. ein Wärmetauscher der der Konditionierungseinheit und/oder mind. ein Wärmetauscher der Trenneinheit wärmetauschen eingebunden ist..

## Beschreibung

Die Erfindung betrifft eine Anlage und ein Verfahren zur kontinuierlichen, katalytischen Hydrierung von MDA, insb. zur Herstellung von Methylenbis(cyclohexylamin), wie insb. 4,4`-Diaminodicyclohexylmethan (PACM).

Verfahren zur Hydrierung organischer Verbindungen, insbesondere zur Hydrierung von aromatischen Verbindungen zu den entsprechenden Cyclohexan-Derivaten sind aus dem Stand der Technik bereits bekannt.

Methylenbis(cyclohexylamin) ist ein bei Standardbedingungen (SATP) festes oder flüssig vorliegendes cycloaliphatisches Amin, das üblicherweise über die Flüssigphasen-Hydrierung von MDA hergestellt wird. Das Akronym MDA wurde historisch als Abkürzung für das bei der Umsetzung von Anilin und Formaldehyd gebildete, vor allem "Methylendianilin" (Diaminodiphenylmethan) umfassende Produktgemisch eingeführt und wird weiterhin zur Bezeichnung des mittlerweile großtechnisch erzeugten Verfahrensproduktes verwendet. Das Produkt der Hydrierung, das vor allem Methylenbis(cyclohexylamin) umfasst, wird deswegen oft auch als H12MDA bezeichnet.

MDA ist aufgrund seines Herstellungsprozesses üblicherweise ein Gemisch aus verschiedenen Diaminodiphenylmethanen. Vor allem besteht es aus 4,4`-Diaminodiphenylmethan. Es können jedoch auch 2,4`- und 2,2`-Isomere vorliegen. MDA kann weiterhin bei der Umsetzung von Anilin und Formaldehyd gebildete Reaktionsprodukte mit drei oder mehr aromatischen Ringen, insbesondere solche mit drei oder mehr Phenylringen, enthalten. Diese Reaktionsprodukte mit drei oder mehr aromatischen Ringen werden auch als Mehrkernverbindungen bezeichnet.

Bei dem im Handel erhältlichen Methylenbis(cyclohexylamin) handelt es sich aufgrund des hohen Anteils an 4,4`-Diaminodiphenylmethan im eingesetzten MDA größtenteils um 4,4`-Diaminodicyclohexylmethan bzw. Bis(para-Aminocyclohexyl)methan. Aufgrund der potentiellen Anwesenheit der entsprechenden 2,4'- und 2,2`-Diaminophenylmethan-Isomere im MDA kann in Methylenbis(cyclohexylamin) auch 2,4`-Diaminodicyclohexylmethan und 2,2`-Diaminodicyclohexylmethan vorliegen. Weiterhin kann hydriertes MDA neben Methylenbis(cyclohexylamin) weiterhin noch (ggf. partiell) hydrierte Mehrkernverbindungen enthalten.

US 5,578,546 A offenbart, dass 1947 erstmalig ein Verfahren zur Herstellung von Methylenbis(cyclohexylamin) beschrieben und 1965 in den technischen Maßstab überführt wurde. Die Hydrierung von MDA ist stark exotherm. So gibt WO 2010/069484 A1 eine Reaktionsenthalpie von -1600 kJ/mol an.

In Folge der Hydrierung werden je nach Verfahren verschiedene Diastereoisomere gebildet. Dabei kann das von 4,4`-Diaminodiphenylmethan abgeleitete Produkt 4,4`-Diaminodicyclohexylmethan (PACM) als trans/trans-, cis/cis- und cis/trans-Isomer vorliegen und ist deswegen in der Regel ein Gemisch dieser Isomere mit unterschiedlichen Anteilen. Mit steigendem trans/trans-Gehalt steigt dabei der Schmelzpunkt der Verbindung. Deshalb unterscheiden sich die Einsatzgebiete je nach Isomerengehalt stark: Während Methylenbis(cyclohexylamin)-Qualitäten mit niedrigem trans/trans - Gehalt (z.B. 10-30 Gew.-%) im Bereich der Amin- und Isocyanat-Vernetzer, insbesondere im Bereich von Zwei-Komponenten-Harzen Einsatz finden, finden Qualitäten mit hohem trans/trans - Gehalt (z.B. ≥ 48 Gew.-%) vor allem Anwendung als Regulator in Polyamidverbindungen. Gerade die Herstellung von Produkten mit niedrigem trans/trans-Gehalt stellt eine Herausforderung dar, da das thermodynamische Gleichgewicht, wie in US 3,636,108 A beschrieben wird, im Bereich wesentlich höherer trans/trans-Anteile (bis zu 51,2 %) liegt. US 2,606,925 A zeigt zudem, dass das Gleichgewicht nachträglich durch längeres Temperieren in Richtung eines höheren Anteils an trans/trans-Isomer verschoben werden kann.

Die Zusammensetzung des Hydrierungsproduktes hängt auch von der Zusammensetzung des eingesetzten MDAs ab: Oft wird MDA in Qualitäten von MDA50 bis MDA100 eingesetzt, wobei die zwischen 50 und 100 liegende Zahl den Gehalt an Diaminodiphenylmethanen im MDA-Gemisch angibt. MDA50 ist dabei eine MDA-Qualität, die wie oben erläutert prozessbedingt etwa 50 Gew.-% Diaminodiphenylmethanen und 50 Gew.-% Mehrkernverbindungen enthält. Die einzelnen Mehrkernverbindungen können entsprechend der enthaltenen Aromatenanzahl als 3-Kern-Verbindungen, 4-Kern-Verbindungen, etc. bezeichnet werden. MDA50 ist dabei die am meisten produzierte Qualität und wird hauptsächlich zu Methylendicyclohexyldiisocyanat (MDI) weiterverarbeitet. MDA100 ist reines MDA bzw. Diaminodiphenylmethan ohne Mehrkernverbindungen. MDA85 oder MDA90 sind andere auf dem Markt verfügbare Qualitäten mittlerer Reinheit. Wenn in Patentschriften zum Herstellverfahren von Methylenbis(cyclohexylamin) auf die Reinheit der MDA-Qualität eingegangen wird, so ist meist von MOA 100 die Rede (z.B. CN 110204447 B). Dagegen stellt US 2005/261525 A1 bevorzugt auf die Hydrierung von MDA50 ab. Die dabei als Hochsieder anfallenden hydrierten, oligomeren Amine eignen sich als Vernetzer mit besonders niedrigen Dampfdrücken für eine Reihe von Spezialanwendungen, wie US 2004/162409 A1 zeigt.

Der Gehalt an (ggf. partiell) hydrierten Mehrkernverbindungen im Produkt nimmt in der Reihenfolge der Edukte MDA50, MDA85, MDA90, MDA100 ab, da der Gehalt an Mehrkernverbindungen von MDA50 zu MDA100 abnimmt.

Aus der WO 2009/153123 A1 ist ein kontinuierliches Verfahren und einen Reaktor zur Hydrierung organischer Verbindungen in einem mehrphasigen, mehrstufigen System in Gegenwart eines homogenen oder heterogenen Katalysators bekannt. Als Katalysatoren werden u.a. Edelmetalle, wie Platin, Palladium, Ruthenium und Rhodium oder andere Übergangsmetalle, wie Molybdän, Wolfram und Chrom vorgeschlagen. Hierbei können die heterogenen Katalysatoren auf Trägermaterialien angeordnet sein, wie bspw. Kohlenstoff, Aluminiumoxid, Siliciumdioxid, Zirkoniumdioxid, Zeolithe, Alumosilikate oder Mischungen dieser Trägermaterialien. Als Substrate werden in diesem Verfahren bevorzugt aromatische Verbindungen enthaltend Amino-Substituenten eingesetzt, beispielsweise MDA, Polymer-MDA, Anilin, 2,4-Diaminotoluol, 2,6-Diaminotoluol, o-Phenylendiamin, etc. Die heterogenen Katalysatoren werden in Suspension eingesetzt.

DE 19533718 A1 offenbart ein Verfahren zur Hydrierung von aromatischen Verbindungen, in denen mindestens eine Aminogruppe an einen aromatischen Kern gebunden ist. Dazu kann ein heterogener Katalysator enthaltend Ruthenium und gegebenenfalls mindestens ein Metall der I-VII- oder VIII-Nebengruppe verwendet werden. Als Trägermaterial wird beispielsweise Aluminiumoxid, Siliciumoxid, Titanoxid oder Zirkoniumoxid, vorzugsweise Aluminiumoxid oder Zirkoniumoxid, eingesetzt. Als Beispiel angegeben wird lediglich ein Katalysator enthaltend Ruthenium auf dem Trägermaterial Aluminiumoxid, nicht jedoch Zirkoniumoxid.

EP 1337331 A1 offenbart ein Verfahren zur katalytischen Hydrierung von aromatischen oder heteroaromatischen Aminen, wobei als Aktivmetall Ruthenium fungiert und der Katalysator mindestens ein weiteres Metall der I-, VII-, oder VIII-Nebengruppe enthält und diese auf einem Trägermaterial aufgebracht sind. Hierbei werden als aromatische Verbindungen u.a. 4,4'-MDA und Isomeren hiervon eingesetzt. Auch die EP 0111238 A1 offenbart ein Verfahren zur katalytischen Hydrierung von 4,4'-MDA, dadurch gekennzeichnet, dass die Hydrierung in Gegenwart von geträgertem Ruthenium in Anwesenheit von Nitraten und Sulfaten der Alkalimetalle und Nitraten der Erdalkalimetalle erfolgt. Ein vergleichbares Verfahren wird in der EP 1366812 A1 offenbart, wobei als Trägermaterialien unter anderem Aluminiumoxid, Siliciumoxid, Titanoxid und Zirkoniumoxid genannt werden.

Weitere Verfahren zur Hydrierung organischer Verbindungen offenbaren WO 2011/003899 A1 und WO 2009/090179 A1. Hier werden Verfahren zur Hydrierung von aromatischen Aminen mit Wasserstoff in Gegenwart eines Ru-Katalysators, welcher, u.a. Zirkoniumoxid-Trägermaterial enthält, offenbart.

Schließlich ist aus der EP 2 883 863 B1 ein Verfahren und eine Anlage zur Hydrierung von 4,4'-Methylendianilin (MDA) und/oder Polymer-MDA mit Wasserstoff in Gegenwart eines Katalysators bekannt. Hierbei wird als Katalysator Ruthenium vorgeschlagen, welcher auf einem Zirkoniumoxid-Trägermaterial aufgebracht ist. Bezüglich des Reaktors verweist die EP 2 883 863 B1 auf den Reaktor bzw. das Reaktorkonzept der WO 2008/015135 A1. Aus dieser WO 2008/015135 A1 ist ein kontinuierliches Verfahren und eine Anlage zur Hydrierung von Diisononylphtalat zu 1,2-Cyclohexandicabonsäurediisononylester (DINCH) bekannt, wobei DINP als Gemisch in einem organischen Lösungsmittel, mit Wasserstoff bei einem Druck von bis zu 325 bar hydriert wird. Hierbei wird eine in Reihenschaltung von zwei Festbettreaktoren mit jeweils einer immobilen Festbettschüttung vorgeschlagen. Zur Ableitung der Reaktionswärme aus den beiden Reaktoren wird vorgeschlagen, einen Teilstrom des Stoffgemisches nach dem zweiten Festbettreaktor zu rezirkulieren und diesen hierbei zu kühlen. Ein vergleichbares Reaktorkonzept von in Reihe geschalteten Festbettreaktoren zur Hydrierung von ist auch aus der EP 1 566 372 B1 bekannt.

Nachteilig an dem Konzept ist, dass die Kreislaufführung eines Produktteilstroms zu einer erhöhten Bildung von unerwünschten Nebenprodukten führen kann und die Anlagenleistung senkt, wobei erhöhte Energiekosten für die Rückführung und Kühlung des Recyclingstroms erzeugt werden.

Wie ausgeführt, ist der Bedarf an bspw. PACM mit unterschiedlichen Anteilen der jeweiligen Isomeren abhängig vom dem Einsatzzweck bzw. den nachfolgenden Produkten. So sind beispielsweise PACM-Qualitäten mit niedrigem trans/trans-Gehalt von 10 bis 30 Gew.% im Bereich der Amin- und Isocyanat-Vernetzer bevorzugt, insbesondere im Bereich der Formulierung von 2-Komponenten-Harzen, wobei PACM-Qualitäten mit hohem trans/trans-Gehalt von über 48 Gew.% vor allem Anwendung als Regulator in Polyamidverbindungen finden. Die genannten Gew.% beziehen sich hierbei auf das PACM-Isomerengemisch als solches. Gerade die Herstellung von Produkten mit niedrigem trans/trans-Gehalt stellt eine verfahrenstechnische Herausforderung dar, da das thermodynamische Gleichgewicht, wie in US 3,636,108 A beschrieben wird, im Bereich wesentlich höherer trans/trans-Anteile von bis zu 51,2 Gew.% liegt. Weiterhin ist aus der US 2,606,925 A bekannt, dass das Gleichgewicht der PACM-Isomere durch längeres Temperieren nachträglich in Richtung zu einem höheren Anteil an trans/trans-Isomer verschoben wird.

Aufgabe der vorliegenden Erfindung ist es somit, eine verbesserte Anlage und einen verbesserten Prozess bereitzustellen, der hinsichtlich Produktumsatz und Energieeffizienz verbessert ist und insb. die Herstellung definierter Anteile der jeweiligen Isomere im Isomerengemisch ermöglicht.

Die Aufgabe wird erfindungsgemäß mit einer Anlage nach den Merkmalen des Anspruch 1 und einem Verfahren nach den Merkmalen des Anspruchs 12 gelöst.

Hierbei wird eine Anlage vorgesehen, zur kontinuierlichen, katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
umfassend eine Konditionierungseinheit (104) für die Edukte, eine Reaktoreinheit (102) und eine Trenneinheit (106), wobei
- die Konditionierungseinheit (Zu-)Leitungen für Edukte1, Edukt2 und mind. ein Lösungsmittel, mind. einen Wärmetauscher in mind. einer (Zu-)Leitung, mind. einen Mischer zur Mischung der Edukte und/oder mind. eines Edukts mit mind. einem Lösungsmittel umfasst;
- die Reaktoreinheit mind. einen Festbettreaktor als Hauptreaktor mit einer immobilen Katalysatorpackung umfasst, wobei der mind. eine Hauptreaktor
   - einen ersten Strömungsweg für das Stoffgemisch
      über die immobile Katalysatorpackung und

   - einen weiteren hiervon getrennten, geschlossenen Strömungsweg für ein Wärmetauschmedium außerhalb der Katalysatorpackung umfasst, und
      wobei in den Medienumlauf ein Wärmetauscher eingebunden ist;
- die Trenneinheit mind.
- eine erste Trennstufe zur Abtrennung des Lösungsmittels und
- eine zweite Trennstufe zur Abtrennung vom mind. einem Edukt und/oder mind. einem Nebenprodukt vom Produkt umfasst, wobei
   i) ein Sammelkreislauf für einen geschlossenen ersten Medienumlauf umfasst ist, in den als Wärmequelle/n mind. der Wärmetauscher des Hauptreaktors und/oder mind. ein Wärmetauscher der Trenneinheit, sowie ein erster Verdampfer eingebunden sind,
   ii) ein Zwischenkreislauf für einen zweiten geschlossenen Medienumlauf umfasst ist, in den der erste Verdampfer, mind. ein Verdichter und ein zweiter Verdampfer eingebunden sind, und wobei
   iii) mind. ein Verteilerkreislauf für einen weiteren geschlossenen dritten Medienumlauf umfasst ist, in den als Wärmesenke mind. ein Wärmetauscher der Reaktoreinheit, mind.
   ein Wärmetauscher Konfektionierungseinheit und/oder mind. dein Wärmetauscher der Trenneinheit wärmetauschen eingebunden sind.

Hierbei umfasst
- der Sammelkreislauf einen zum ersten Verdampfer hinführenden oder Energie sammelnden Leitungsast und einen rückführenden Leitungsast,
- der Zwischenkreislauf einen zum zweiten Verdampfer hinführenden Leitungsast, auch Dampfleitung genannt, sowie einen vom zweiten Verdampfer zum ersten Verdampfer rückführenden Leitungsast, auch Rückleitung genannt, und
- der Verteilerkreislauf mindestens einen hinführenden Leitungsast (Kopf- oder Dampfleitung) vom zweiten Verdampfer zu dem mind. einen als Wärmesenke eingebundenen Wärmetauscher der Trenneinheit und/oder der Reaktoreinheit zum zweiten Verdampfer.

Vorliegend ist unter einem geschlossenen Kreislauf zu verstehen, dass das jeweilige Wärmetauschmedium nur in diesem Kreislauf zirkulieren kann, insb. nicht in den benachbarten Kreislauf weitergeleitet wird, so dass der Energieaustausch mit dem jeweils gekoppelten Nachbarkreislauf über einen indirekten Wärmetransport zwischen den zirkulierenden (Kreislauf-)Medien erfolgt, ohne Austausch der jeweiligen (Kreislauf-)Medien. Der Zwischenkreislauf ist hierbei gekoppelt mit dem Sammelkreislauf und dem mind. einen Verteilerkreislauf. Der Sammelkreislauf dient als Wärmequelle für den Zwischenkreislauf, der Zwischenkreislauf dient zum Heben des Temperaturniveaus und als Wärmequelle für den Verteilerkreislauf und die dort eingebundenen als Wärmesenke fungierenden Wärmetauscher, insb. die Wärmetauscher der Trenneinheit.

Der von dem Trennkessel kommende flüssige Stoffstrom wird über eine Leitung zur ersten Trennkolonne innerhalb der ersten Trennstufe der Trenneinheit geleitet. Vorteilhafterweise kann bei einer Ausführungsform der Anlage vorgesehen sein, dass in der Leitung vom Trennkessel zur ersten Kolonne eine Entspannungseinheit vorgesehen ist. Hierdurch kann die Reaktoreinheit auf einem ersten, hohen Druckniveau betrieben werden und die ersten Trennstufe der Trenneinheit auf einem zweiten, tieferen Druckniveau.

Die Anlage dient vorzugsweise zur kontinuierlichen, katalytischen Herstellung von Methylenbis(cyclohexylamin) als Produkt, insb. zur Produktion von 4,4'-Diaminodicyclohexylmethan (PACM), nach der Formel (I)

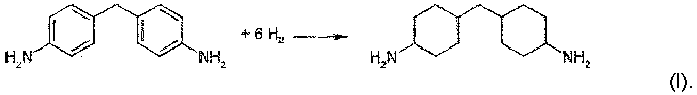

Mit dem separaten Nachreaktor, insb. dem adiabatischen Nachreaktor, ist eine optimierte Steuerung des Prozesses und der Anlage möglich geworden, wodurch die Selektivität durch vom Hauptreaktor (Auslass) abweichende Eintrittstemperatur des Nachreaktors möglich wird. Typischerweise kann die Eintrittstemperatur des Nachreaktors bei der gleichen oder einer geringfügig niedrigeren Temperatur vorgesehen werden, die bis zu 30 °C unterhalb der Auslasstemperatur des Hauptreaktors liegen kann. Somit kann mind. zeitweise eine Temperaturerhöhung im (adiabatischen) Nachreaktor von 20 °C bis 40 °C, typischerweise von 20 °C bis 30 °C zugelassen werden, und so gezielt die gewünschte Produktqualität (Isomerenverhältnis) eingestellt werden. Auf diese Weise ist ein sehr niedriger und ein sehr genauer Anteil an trans/trans-Isomeren in dem Isomerengemisch einstellbar. Der Hauptreaktor kann auf einem möglichst niedrigen Temperaturniveau betrieben werden, so dass der trans/trans-Anteil des PACM im Stoffstrom (Auslass des Hauptreaktors) ca. 13 bis 20 Gew.% beträgt, wobei ca. 13 % bei einem neuen bzw. regenerierten Katalysator erreichbar sind und 20 Gew.% bei einem Katalysator nach Langzeitnutzung (kurz vor Austausch/Regeneration). Auch wenn der Hauptreaktor vorliegend als "isotherm/-isch" bezeichnet wird, wird dieser ideale Zustand bei industrieller Anwendung nur bedingt erreicht, so dass sich innerhalb des Hauptreaktors wegen unvollständigem Wärmeabtransport ein Temperaturgradient von ca. 5 bis 10 °C in radiale und auch in Strömungsrichtung ausbildet. Abhängig von der Phase, in der sich der Hauptreaktor befindet, kann es mind. zeitweise sinnvoll sein, wenn die Eintrittstemperatur des Nachreaktors um 5 bis 20 °C höher als die des Hauptreaktors ist.

Der Nachreaktor wird über den vorlaufenden Wärmetauscher mit einer etwas höheren Temperatur derart beschickt, dass die gewünschte restliche Reaktion und Isomerenumformung zu dem finalen, gewünschten trans/trans-Verhältnis von bspw. 17 bis 23 Gew.% erfolgt. Der Nachreaktor mit der dortigen schwach exothermen, restlichen Reaktion wird hierbei weitgehend adiabat betrieben, was aber nicht im idealen Sinne zu verstehen ist. Im Nachreaktor wird der Isomerengehalt an trans/trans-Isomeren weiter erhöht wird, um dabei MDA vollständig umzusetzen. Vorteilhafterweise ist die Konzentration von MDA stromabwärts zum Nachreaktor kleiner 1000 ppm. Bei einer bevorzugten Verfahrensführung, wird eine trans/trans-Anteil im Isomerengemisch von 10 bis 20 Gew.% nach dem Hauptreaktor erreicht, ca. 13 Gew.% bei dem frischen, aktiveren Katalysator und bis zu ca. 20 Gew.% bei einem (alten) beladenen Katalysator.

Mit fortschreitender Zeit erfolgt ein Absinken der Katalysatoraktivität bis ein Austausch oder eine Regenerierung erforderlich ist. Hierzu wird parallel durch Regelung des Kühlkreislaufs die Betriebstemperatur angehoben, d.h. eine geringere Kühlung über den im Kühlkreislauf eingebundenen Wärmetauscher vorgenommen, um Umsatz und Selektivität insgesamt auf einem weitgehend gleichbleibenden Niveau zu halten. Dies hat zur Folge, dass das Isomerenverhältnis verschoben wird, hin zu höheren trans/trans-Anteilen im Produkt. Hierbei hat es sich als sehr vorteilhaft erwiesen, dass die Temperatur im Feed des Nachreaktors über den vorlaufenden Wärmetauscher eigenständig geregelt werden kann, insb. gleichläufig. So wird mit steigender Betriebstemperatur des Hauptreaktors über die Nutzungszeit des Katalysators die Feedtemperatur im Stoffstrom des Nachreaktors abgesenkt.

Vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, kann die Betriebstemperatur des Hauptreaktors erhöht und Temperatur im Stoffstrom im Einlass (Feed) des Nachreaktors gleichgehalten oder gesenkt werden. Vorteilhafterweise ist die Anlage so ausgebildet, dass die Temperaturerhöhung oder -Absenkung kontinuierlich und/oder schrittweise erfolgen kann.

Der Hauptreaktor wird hierbei isotherm oder weitgehend isotherm und der Nachreaktor adiabat oder weitgehend adiabat betrieben. Zum Zeitpunkt t₀ dem Verfahrensstart kann neben dem Hauptreaktor auch der Katalysator im Nachreaktor erneuert oder regeneriert worden sein. Vorteilhafterweise wird der Zyklus zur Regeneration des Nachreaktors eigenständig und vom Hauptreaktor unabhängig bestimmt, insb. wenn o.g. Zusammenwirken der beiden Reaktoren nicht mehr die Produktion des gewünschten niedrigen trans/trans-Anteils im PACM ermöglicht.

Vorteilhafterweise ist die Mischereinheit zweiteilig ausgebildet und umfasst bspw. einen Mischapparat und einen Gas-Sättiger. Der Mischapparat kann insb. ein dynamischer oder statischer Mischer sein, der zur innigen Verbindung von MDA (Edukt1) mit dem Lösungsmittel geeignet ist. Der Gas-Sättiger kann insb. eine kleine Kolonne oder ein Kessel sein, in dem geeignete Einbauten vorhanden sind, um das unter einem hohen Druck zugeführte H2-Gas, intensive im MDA-Lösungsmittel-Stoffstrom zu lösen und/oder homogen vor dem Eintritt in den Hauptreaktor zu verteilen. Der H2-Gasdruck beträgt vorteilhafterweise 70 bar bis 100 bar.

Mit dem Begriff "immobile Katalysatorpackung" oder "immobiler Katalysator" ist jede Form eines lokalen, nicht mit dem Stoffstrom strömenden oder fließenden Katalysator gemeint, wie insb. Katalysatorschüttungen (Pellets oder beschichtete Trägerkörper) oder feste Einbauten, die mit Katalysatormaterial beschichtet sind. Vorteilhafte Einbauteile, die eine Katalysatorbeschichtung aufweisen, können bspw. Gittern, Platten oder sonstigen Köper sein, die im Hauptreaktor angeordnet sind.

Im vorliegenden Zusammenhang meint eine "XY-einheit" und/oder "XY-stufe" immer auch mind. einen entsprechenden Apparat, Vorrichtung oder dergleichen, der/die von der jeweiligen Einheit oder Stufe umfasst ist. So meint bspw. eine Mischeinheit/-stufe, dass diese mind. einen Mischer/- vorrichtung umfasst.

Im vorliegenden Zusammenhang meint "Wärmetausch" oder "Wärmetauscher" immer einen indirekten Wärmetausch und entsprechende Bauformen mit geschlossenen Stoff- und Medienführungen, es sei denn, es ist ausdrücklich etwas hiervon Abweichendes beschrieben.

Vorliegend werden im Wesentlichen folgende Energiekopplungen (EK) betrachtet, wie eine integrierte Energiekopplung oder eine direkte Energiekopplung, wobei die integrierte EK eine integrierte Energiekopplung meint, unterteilt in
a. eine integrierte stoffbasierte Energiekopplung (integrierte stoffbasierte EK) von mind. zwei Stoffströmen in einem Wärmetauscher im indirekten Wärmeaustausch meint, d.h. eine bauliche Integration in einem einzigen Wärmetauscher (Apparat), d.h. wo vormals zwei Wärmetauscher vorgesehen waren, sind beide Wärmetransportaufgaben einer baulichen Einheit (Wärmetauscher) integriert,
b. eine integrierte medienbasierte Energiekopplung (integrierte medienbasierte EK) von mind. zwei Wärmetauschmedien in einem Wärmetauscher im indirekten Wärmeaustausch meint, d.h. eine bauliche Integration in einem einzigen Wärmetauscher (Apparat);

Die vorgenannten EK können als direkte Energiekopplung (direkte EK) ausgestaltet sein, indem eine serielle EK oder serielle Verschaltung von mind. zwei Wärmetauschern vorgesehen ist.

Mit der "Kondensationseinheit" der ersten Trennstufe ist ein einzelner Wärmetauscher oder eine Gruppe von Wärmetauschern bezeichnet, die zur mind. teilweisen Kondensation und/oder Abkühlung der über die Kopfleitung/en abgeleiteten Anteile an Leichtsiedern dienen. Eine derart bezeichnete "Kondensationseinheit" muss keine (geschlossene) Baueinheit darstellen. Somit wird zum Teil der Begriff "Kondensationseinheit" und einen einzelner "Wärmetauscher" auch synonym verwendet.

Die als "erste Trennstufe" bezeichnete Stufe ist insb. dadurch bestimmt und weist entsprechende Apparate und Leitungen auf, dass das Lösungsmittel (gezielt) vom produktreichen Stoffstrom abzutrennen und vorteilhafterweise auch zur Verwendung in die Reaktoreinheit und/oder die Konditionierungseinheit zurückgleitet wird. Die erste ersten Trennstufe ist dadurch bestimmt, dass mind. 80%, idealerweise wird mind. 90 % des Lösungsmittels abgetrennt wird. In analoger Weise meint die als "zweite Trennstufe" bezeichnete Stufe der Trenneinheit, dass diese dadurch bestimmt ist und entsprechende Apparate und Leitungsführungen aufweist, um das Produkt von Nebenprodukten und Edukten, insb. MDA zu trennen und das Produkt aufzureinigen. Hierbei sind die erste und zweite Trennstufe ggf. nicht ganz streng getrennt und es kann ein Überlappungsbereiche oder ein Apparat im Übergangsbereich vorhanden sein, in dem sowohl Lösungsmittel als auch mind. ein Nebenprodukt oder mind. ein Edukt vom Produkt getrennt wird. In dem vorliegenden Zusammenhang meint die "Abtrennung von Lösungsmittel" in der ersten Trennstufe und "Abtrennung von mind. einem Edukt und/oder mind. einem Nebenprodukt vom Produkt", wobei das Produkt insb. PACM sein kann, wobei die Abtrennung keine absolute Abgrenzung der Trennstufen meint, sondern jeweils "im Wesentlichen" nur den/die genannten Stoff/e betrifft.

Der von einem Druckentspannungstrennkessel, nachstehend Trennkessel genannt (auch teilweise "Flashbehälter" genannt), kommende flüssige Stoffstrom wird über eine Leitung zur ersten Trennkolonne innerhalb der ersten Trennstufe der Trenneinheit geleitet. Der Trennkessel zeichnet sich dadurch aus, dass durch Entspannung (Druckabsenkung) der zufließende Stoffstrom in eine Dampfphase (Lösungsmittel, lösungsmittelreich) und eine Flüssigphase (verarmt an Lösungsmittel) aufgetrennt wird und im Regelbetrieb im Trennkessel beide Phasen vorliegen. Der Trennkessel kann zusätzlich einen Sumpfumlauf mit integriertem Wärmetauscher aufweisen oder hieran angeschlossen sein, um durch Erwärmung der Flüssigphase den abtrennbaren Dampfanteil über den druckbedingten Anteil hinaus zu erhöhen. Weiterhin kann ein Trennkessel Einbauten oder Füllkörper umfassen, insb. um das Mitreißen von nicht oder nur unvollständig an Lösungsmittel verarmten Tröpfchen zu verhindern. Vorteilhafterweise kann bei einer Ausführungsform der Anlage vorgesehen sein, dass in der Leitung vom Trennkessel zur ersten Kolonne eine Druckregeleinheit vorgesehen ist. Hierdurch kann die Reaktoreinheit auf einem ersten, hohen Druckniveau betrieben werden und die ersten Trennstufe der Trenneinheit auf einem zweiten, tieferen Druckniveau. Im vorliegenden Zusammenhang meint also Trennkessel (Flashbehälter) einen Apparat, indem im Wesentlichen durch Druckentspannung eine Phasentrennung veranlasst wird. Trennkolonne meint vorliegend hingegen einen Apparat, in dem im Wesentlichen durch Energiezufuhr einen Trennung in eine Dampfphase und Flüssigphase erfolgt, insb. unter Einbindung eines Kopfumlaufs, indem im Kopf mind. ein Teil der auskondensierten Flüssigkeit zurück in die Kolonne geleitet wird.

Der Begriff "Eduktmischung" meint in dem vorliegenden Zusammenhang die Mischung aus Stoffen, die beim Eintritt in den (ersten) Hauptreaktor vorliegt, also die Mischung aus allen Edukten, Lösungsmitteln, Hilfsstoffen etc. In und nach dem mind. einen Reaktor wird die strömende Mischung aus Stoffen in jedem Grad der Reaktion oder nachfolgenden Aufreinigung als "Stoffstrom" oder "Stoffmischung" (auch "Stoffgemisch") bezeichnet, wobei zum Teil adjektivische Beschreibungen wie "produktreich" oder "lösungsmittelreich" angefügt sind. Die stoffliche Zusammensetzung des Stoffstroms an jedem Ort der Anlage ergibt sich für den Fachmann allerdings auch in naheliegender Weise durch ebendiesen Anlagenort und stromaufwärts befindliche Anlagenkomponenten und insb. die verfahrenstechnischen Apparate der Anlagen. Die Reinstoffe, wie bspw. das Produkt PACM, sowie die Nebenprodukte LB und HB, sind gesondert benannt und ausgewiesen. Hierbei steht "LB" für "Low Boiler", einem werthaltigen Stoffgemisch, das gesondert aus dem Stoffstrom und vom Produkt abgetrennt wird und einen niedrigen Siedepunkt von ca. 240 °C bis 290 °C aufweist. In analoger Weise steht "HB" für "High Boiler", einem werthaltigen Stoffgemisch, das gesondert aus dem Stoffstrom und vom Produkt abgetrennt wird und einen hohen Siedepunkt von > 350 °C aufweist.

Vorliegend wird insb. eine Anlage und ein Verfahren zur Produktion von Methylenbis(cyclohexylamin) als Produkt, insb. zur Produktion von 4,4'-Diaminodicyclohexylmethan (PACM), durch katalytische Hydrierung von Methylendianilin beansprucht. Hierbei ist das primäre Produkt PACM, das aus 4,4`-Diaminodiphenylmethan (4,4'-MDA; primärer Anteil des Edukts1) hydriert wird, insb. mit dem genannten niedrigen trans/trans-Isomerenverhältnis, so dass die Anlage und das Verfahren insb. zur Produktion von 4,4'-Diaminodicyclohexylmethan (PACM) durch kontinuierliche, katalytische Hydrierung von 4,4'-Diaminodiphenylmethan (4,4'-MDA) dient und geeignet ist. Die weiterhin vorliegenden Anteile 2,4'-MDA und 2,2'-MDA des MDA werden mind. zu geringen Anteilen parallel zu Reaktionsprodukten gewandelt, wobei diese in der Regel in der Produktmischung verbleiben. Weiterhin können vorteilhafterweise ebenfalls durch dieses Verfahren bzw. diese Anlage in der zweite Trennstufe der Trenneinheit sonstige Nebenprodukte aufgereinigt und abgetrennt werden, insb. in mind. einer Trennkolonne. Diese stellen regelmäßig sekundäre (werthaltige) Produkte dar und sind hierin im Wesentlichen als High Boiler (HB) und Low Boiler (LB) beschrieben und gemeint. Vorteilhafterweise ist das Lösungsmittel aus der nachfolgenden Gruppe von Stoffen: Cyclohexan, Dioxan, Tetrahydrofuran (THF), Cyclohexylamin, dicyclohexylamin, Methanol, Ethanol, Isopropanol, n-Butanol, 2-Butanol, 2-Methoxy-2-methylpropan (MTBE), Methylcyclohexan oder ein Gemisch hieraus. Vorteilhafterweise wird das Lösungsmittel, insb. THF im Überschuss zum MDA zugeführt, so dass vorteilhafterweise das Verhältnis der Massenströme von Lösungsmittel, insb. THF, zu MDA am Einlass des Hauptreaktors im Bereich von 1,0 bis 8,0 liegt, insbesondere im Bereich von 2,0 bis 6,0.

Bei einer vorteilhaften Ausführungsform der Anlage kann vorgesehen sein, dass im Zwischenkreislauf ein Wärmetauscher kreuzweise in den hinführenden Leitungsast (Dampfleitung) und den rückführenden Leitungsast eingebunden ist, um die Temperatur im hinführenden Leitungsast anzuheben.

Vorteilhafterweise ist der Wärmetauscher mit einem ersten Innenraum, insb. dem Dampfraum, in dem hinführenden Leitungsast zwischen dem ersten Verdampfer und dem mind. einen, insb. dem ersten, Verdichter wärmetauschend eingebunden, wobei der rückführende Leitungsast in den zweiten Innenraum des Wärmetauscher, insb. innerhalb der WT-Rohre/-Rohrbündel, eingebunden ist. Anders ausgedrückt, strömt das dampfförmige Medium der hinführenden Leitung außerhalb der bspw. Wärmetauscherrohre des Wärmetauschers und das weitgehend kondensierte, flüssige Medium des rückführenden Leitungsastes strömt innerhalb der bspw. Wärmetauscherrohre.

Bei einer Ausführungsform kann es vorteilhaft sein, wenn eine (Quer-)Leitung zwischen hinführendem und rückführendem Leitungsast vorgesehen ist, die aus der rückführenden Leitung an einem Leitungsknoten abzweigt und an einem Leitungsknoten in den hinführenden Leitungsast einleitet, wobei die Abzweigung vorteilhafterweise zwischen dem Wärmetauscher und dem ersten Verdampfer, insb. vor dem Entspannungsventil angeordnet ist und die Einleitung vorteilhafterweise entweder zwischen
- dem Wärmetauscher und dem mind. einen Verdichter oder
- zwischen zwei Verdichtern, die in dem hinführenden Leitungsast zur Druckerhöhung eingebunden sind.

Der Vorteil der kreuzweisen Verschaltung durch den Wärmetauscher ist, dass die Temperatur in dem hinführenden Leitungsast durch das rückströmende Medium um 2 bis 10 °C angehoben wird. Diese Verschaltung dient der leichten Überhitzung des hinführenden Leitungsastes, um Kondensatbildung im Zulauf des Verdichters zu verhindern, wodurch diese geschädigt werden könnte. Mittels der Quereinleitung von flüssigem Medium über eine Druckregeleinheit aus der rückführenden Leitung in die hinführende Leitung stromabwärts zu einem Verdichter, insb. zwischen zwei Verdichter, kann vorteilhafterweise der Volumenstrom und damit die Energiemenge bedeutsam erhöht werden, ohne hierfür den Strom-/Energieverbrauch des vorlaufenden Verdichters zu erhöhen. Das über die Zwischeneinspeisung zugeführte Medium wird an der Druckregeleinheit entspannt und als Dampf eingeleitet. Die Effizienz in dem hinführenden Leitungsast wird durch die Zwischeneinspeisung um ca. 5 bis 10% gesteigert.

Bei einer weiteren vorteilhaften Ausführungsform der Anlage kann vorgesehen sein, dass im Zwischenkreislauf im hinführenden Leitungsast eine Gruppe von mind. zwei Verdichtern eingebunden ist. Es hat sich als vorteilhaft erwiesen, den gewünschten Druckanstieg mehrstufig auszuführen, weil hierdurch insgesamt der Stromverbrauch sinkt, da die nachfolgenden Verdichter auf einen immer geringeren Dampfvolumenstrom einwirken müssen, wodurch sie baulich kleiner ausgeführt werden können.

Bei einer weiteren vorteilhaften Ausführungsform der Anlage kann vorgesehen sein, dass in den Medienumlaufdes Sammelkreislaufs ein Sammeltank und eine Pumpe eingebunden sind, wobei ein Wärmetauscher stromaufwärts zum Sammeltank und/oder auf der Saugseite der Pumpe angeordnet ist. Bei einer Ausführungsform ist der Wärmetauscher in den Sammeltank integriert und/oder der Sammeltank weist eine integrierte Temperiereinheit auf. Der Vorteil hierbei ist, dass unabhängig vom aktuellen Energieeintrag in den Sammelkreislauf und/oder der aktuellen Energieentnahme am eingebundenen ersten Verdampfer, im hinführenden Leitungsast insgesamt das erforderliche Temperaturniveau und der erforderliche Volumenstrom an Medium bereitgestellt werden kann. Das zirkulierte Wärmetauschmedium des Sammelkreislaufs ist vorteilhafterweise Wasser oder eine im Wesentlichen wässrige Lösung.

Bei einer weiteren vorteilhaften Ausführungsform kann vorgesehen sein, dass der Verteilerkreislaufs einen ersten Leitungsast als dampfführende Zuleitung umfasst, wobei in diesen Leitungsast mind. ein Verdichter eingebunden ist, idealerweise eine Gruppen von Verdichtern vorgesehen ist, die zwei bis fünf Verdichter umfasst, insb. zwei oder drei Verdichter umfasst. Zwei oder mehr Verdichter sind vorteilhafterweise zueinander in Serie geschaltet.

Auf diese Weise kann mit hohem Wirkungsgrad die Temperatur des vom zweiten Verdampfer kommenden Dampfes in der hinführenden Leitung um 30 bis 150 °C angehoben werden, insb. um 70 bis 120 °C angehoben werden. Insgesamt ist der Verteilerkreislauf eine Wasserkreislauf, bzw. eine Kreislauf, dessen Wärmetauschmedium Wasser oder eine wässrige Lösung ist.

Bei einer weiteren vorteilhaften Ausführungsform der Anlage kann vorgesehen sein, dass bei zwei oder mehr Verdampfern in dem hinführenden Leitungsast, vom zweiten Verdampfer mind. eine (Sumpf-)Leitung und/oder mind. einen Abzweigung einer (Sumpf-)Leitung auf die Saugseite mind. eines Verdichters führt, wobei in die (Sumpf-)Leitung eine Pumpe eingebunden ist. Vorteilhafterweise ist bei einer Ausführungsform zwischen jedes Paar aus zwei Verdichtern jeweils eine (Sumpf-)Leitung und/oder mind. einen Abzweigung einer (Sumpf-)Leitung geführt. Es hat sich überraschenderweise als insgesamt energetisch vorteilhaft herausgestellt, dass auf einem etwas niedrigeren Temperaturniveau befindliche flüssige Medium der (Sumpf-)Leitung in den hinführenden (Dampf-)Leitungsast einzuspeisen, weil ein stark erhöhter Massenstrom erreicht wird, ohne wesentlich den Energiebedarf des jeweils stromabwärts befindlichen Verdichters zu erhöhen. Vorteilhafterweise ist in der (Sumpf-)Leitung bzw. den jeweiligen Abzweigung von der (Sumpf-Leitung eine Druckregeleinheit vorgesehen, um durch die Entspannung das flüssige Medium aus dem (Sumpf-)Leitung zu verdampfen, so dass die Einspeisung in den hinführenden Leitungsast als dampfförmiges Medium erfolgt. Weiterhin werden über die Druckregeleinheiten die jeweiligen Volumenströme geregelt bzw. bedarfsweise vollständig abgestellt.

Anders beschrieben besteht ein Vorteil darin, dass bei der Zwischeneinleitung zwischen zwei Verdichter auch ein Vorteil für die zweite Verdichterstufe erreicht wird. Nach der ersten Verdichterstufe ist das dampfförmige Medium überhitzt. Diese Überhitzung wird durch Zwischeneinspeisung mit kälterem Dampf reduziert trotz des stofflichen Eintrags, wodurch der Volumenstrom zur zweiten Verdichterstufe reduziert wird, so dass der Stromverbrauch des zweiten Verdichters zur weiteren Verdichtung gesenkt wird.

Bei einer weiteren vorteilhaften Ausführungsform der Anlage kann vorgesehen sein, dass die (Sumpf-)Leitung mind. zwei Äste aufweist, wobei jeder Ast der (Sumpf-)Leitung zwischen zwei der Verdichter geführt ist, und wobei mind. ein Ast der (Sumpf-)Leitung einen Druckregler umfasst, wobei alternativ oder zusätzlich Druck- und/oder Temperatursensoren vorgesehen sein können. Die in der (Sumpf-)Leitung arbeitende Pumpe ist vorteilhafterweise stromabwärts zum zweiten Verdampfer und stromaufwärts zur ersten Abzweigung oder den Leitungsästen für die jeweilige Zwischeneinspeisung angeordnet.

Bei einer weiteren vorteilhaften Ausführungsform der Anlage kann vorgesehen sein, dass in den rückführenden Leitungsast, auch (Rück-)Leitung genannt, des Verteilerkreislaufs ein Wärmetauscher wärmetauschend eingebunden ist. Dieser Wärmetauscher ist stromabwärts zum Leitungsast zur Verteilung in den rückführenden Leitungsast eingebunden. Dieser Wärmetauscher ist ein Bedarf- oder Regelungswärmetauscher, über den sichergestellt werden kann, dass unabhängig vom Energieverbrauch bzw. der Energieabgabe im Leitungsast zur Verteilung, dem zweiten Verdampfer Medium auf dem erforderlichen Temperaturniveau zur Verfügung gestellt wird. Dieser Bedarfs- oder Regelungswärmetauscher ist keinem sonstigen Apparat der Anlage zugeordnet, d.h. ist nicht vorgesehen, einen sonstige Wärmetauschaufgabe zu erfüllen, als den Vorlauf zum zweiten Verdampfer zu regeln.

Bei einer weiteren vorteilhaften Ausführungsform der Anlage kann vorgesehen sein, dass der Verteilerkreislauf einen weiteren Leitungsast zur Verteilung- und/oder Abgabe von Energie und einen weiteren Leitungsast zur Medienrückführung umfasst, wobei in den Leitungsast zur Verteilung mind. ein Wärmetauscher der Reaktoreinheit, mind. ein Wärmetauscher der Konditionierungseinheit und/oder mind. ein Wärmetauscher der Trenneinheit als Wärmesenke eingebunden ist, insb. eine Mehrzahl von den jeweiligen Wärmetauschern eingebunden sind. Bei einer besonders bevorzugten Variante wird jede dieser weiteren Leitungsäste zur Verteilung- und/oder Abgabe von Energie mit jeweils einem Abzweig von dem hinführenden Leitungsast verbunden sind, die mit unterschiedlichen Druckniveaus korrelieren. So kann ein erster Abzweig stromabwärts zum ersten Verdichter, ein zweiter Abzweig stromabwärts zum zweiten Verdichter etc. angeordnet und/oder ein erster Abzweig stromabwärts zu einer ersten Druckregeleinheit, ein zweiter Abzweig stromabwärts zur einer zweiten Druckregeleinheit etc.

Durch diese Gruppierung der Wärmetauscher durch weitere Leitungsäste können einzelne Wärmetauscher oder Gruppen aus zwei oder mehr Wärmetauschern gezielt nach Energiebedarf und nach Bedarf des Temperaturniveaus beschickt werden. So kann vorteilhafterweise ein Wärmetauscher oder eine Gruppe aus Wärmetauschern mit einem niedrigen Temperaturniveau und/oder einer hohen Austauschleitung in Strömungsrichtung zuerst ausgeleitet werden, so dass der Stromverbrauch der nachfolgenden Verdichter entsprechend gesenkt wird. Bedarfsweise kann Medium in dampfförmiger Phase aus der (Sumpf-)Leitung in dem hinführenden Leitungsast zugeführt werden, wie vorstehen beschrieben.

Bei einer weiteren vorteilhaften Ausführungsform der Anlage kann vorgesehen sein, dass bei mehr als einem rückführenden Leitungsast des Verteilerkreislaufs, ein Bedarf- und/oder Regelungswärmetauscher in mind. einem weiteren rückführenden Leitungsast wärmetauschend eingebunden ist, idealerweise in allen rückführenden Leitungsästen ein solcher Bedarf- und/oder Regelungswärmetauscher eingebunden ist.

Bei einer weiteren vorteilhaften Ausführungsform der Anlage kann vorgesehen sein, dass mind. eine Teilzahl der als Wärmesenke eingebundenen Wärmetauscher des Verteilerkreislaufs parallelgeschaltet sind. Vorteilhafterweise sind die einzelnen Wärmetauscher oder die Gruppen aus parallelgeschalteten Wärmetauschern jeweils im Zu- oder Ablauf steuer- und/oder regelbar. Hierbei betrifft die Regelbarkeit insb. die jeweiligen Durchflussmengen an Medium, wobei diese Regelung vorteilhafterweise in Abhängigkeit von dem erforderlichen Temperaturgradienten im jeweiligen Wärmetauscher bzw. der Gruppe von Wärmetauschern und/oder dem erforderlichen Energieübertrag erfolgt. Bei einer weiteren vorteilhaften Ausführungsform ist ein einzelner als Wärmesenke dienender Wärmetauscher bzw. eine Gruppe aus zwei oder mehr Wärmetauschern nicht in den Verteilerkreislauf eingebunden. Die Einbindung dieser Wärmetauscher, insb. (Sumpf-)Wärmetauscher der Trennkolonnen aus der Trenneinheit, hätten bei der Einbindung in den Verteilerkreislaufeinen elektrischen Strombedarf für die Verdichter und/oder einen weiteren Verdichter, der vergleichbar einer direkten, elektrischen Beheizung dieser Wärmetauscher ist. Es hat sich als insgesamt als vorteilhaft erwiesen, wenn bereits bei einem erhöhten Energiebedarf für die direkte, elektrische Beheizung von Wärmetauschern von Faktor 1,2 bis 1,3 gegenüber der Einbindung in den Verteilerkreislauf, aufgrund geringere sonstiger Aufwände, wie eines geringeren Verschleißes, geringerer Stillstandzeiten, etc. eine elektrische Direktbeheizung vorteilhaft ist.

Bei einer weiteren vorteilhaften Ausführungsform der Anlage kann vorgesehen sein, dass die als Wärmequelle wirkenden Wärmetauscher des Sammelkreislaufs in Reihe geschaltet sind. Hierzu sind die eingebundenen Wärmetauscher in Strömungsrichtung des hinleitenden Leitungsastes vorteilhafterweise mit aufsteigendem Temperaturniveau in diesen Leitungsast eingebunden. Als die zentrale Wärmequelle für den Sammelkreislauf dient der im Kühlkreislauf des Hauptreaktors eingebundene Wärmetauscher, der zur Ableitung der exothermen Energie aus der Reaktion dient. Als weitere Wärmequellen dienen insb. die (Kopf-)Wärmetauscher (Kondensatoren) der Trennkolonnen, abhängig von dem dortigen Temperaturniveau im Medium des jeweiligen Wärmetauschers.

Der von dem Trennkessel kommende flüssige Stoffstrom wird über eine (Feed-)Leitung zur ersten Trennkolonne der ersten Trennstufe der Trenneinheit geleitet. Vorteilhafterweise kann bei einer Ausführungsform der Anlage vorgesehen sein, dass in der (Feed-)Leitung vom Trennkessel zur ersten Kolonne eine Entspannungseinheit vorgesehen ist. Hierdurch kann die Reaktoreinheit auf einem ersten, hohen Druckniveau betrieben werden und die erste Trennkolonne der ersten Trennstufe auf einem zweiten, tieferen Druckniveau, wobei der Trennkessel auf einem Zwischenniveau betrieben werden kann.

Weiterhin kann es vorteilhaft sein, wenn stromaufwärts zur ersten Trennkolonne der ersten Trennstufe ein (Vorlauf-)Wärmetauscher vorgesehen ist, insb. auch stromabwärts der Entspannungseinheit bzw. zwischen der Entspannungseinheit und der ersten Trennkolonne. Bei einer besonders vorteilhaften Ausführungsform ist eine Energiekopplung vorgesehen, um den Kondensator stromabwärts zum Trennkessel, einen Kondensator einer Trennkolonne der zweiten Trennstufe oder den (Umlauf-)Wärmetauscher im Medienumlauf des mind. einen Hauptreaktors verschaltet im Wärmetausch mit dem (Vorlauf-)Wärmetauscher der ersten Trennkolonne zu betreiben. Die Energiekopplung kann durch Medienleitung und eine serielle Verschaltung der jeweiligen Wärmetauscher erfolgen oder durch eine integrierte Energiekopplung in einem (baulich) einzigen Wärmetauscher. Die integrierte Energiekopplung hat den Vorteil, sofern räumlich innerhalb der Anlage realisierbar, dass nur ein Temperaturgefälle für den Wärmetransport überwunden werden muss. Durch Energieübertrag kann so der (Feed-)Stoffstrom vor der ersten Trennkolonne der ersten Trennstufe, der nach der stromaufwärtsbefindlichen Druckregeleinheit in der (Feed-)Leitung ein Temperaturniveau von ca. 85 bis 95 °C aufweist, parallel angehoben werden. Da der Hauptreaktor im Laufe des Betriebes bei stetig steigender Temperatur betrieben wird, um die sinkende Katalysatoraktivität zu kompensieren, kann parallel eine stetig steigende Energiemenge an den (Feed-)Stoffstrom vorlaufend der ersten Trennkolonne abgegeben werden. Hierdurch sinkt ebenfalls stetig der Energiebedarf im Sumpfkreislauf, bzw. dem dortig eingebundenen Wärmetauscher der ersten Trennkolonne.

Bei einer Ausführungsform der Anlage, kann es vorteilhaft sein, dass die Reaktoreinheit einen ersten Hauptreaktor und mind. einen stromabwärts befindlichen permanenten, in Serie geschalteten Nachreaktor umfasst. Der besondere Vorteil des Nachreaktors und dessen eingangsseitige Temperierung des Stoffstroms besteht darin, dass auf diese Weise eine optimierte Steuerung der Selektivität der Anteile an Isomeren ermöglicht wird, aufgrund der vom Hauptreaktor abweichenden, bevorzugt höheren Eintrittstemperatur.

Vorteilhafterweise ist der Nachreaktor auch ein Festbettreaktor, bzw. Reaktor mit einem immobilen Katalysator, wie beispielsweise einer Katalysatorschüttung oder mit Katalysator beschichteten Einbauten.

Vorteilhafterweise umfasst der immobile Katalysator Ruthenium, ist mit Ruthenium dotiert oder ist hieraus gebildet. Bei einer vorteilhaften Verfahrensvariante, insb. zur Erreichung eines niedrigen Anteils an trans/trans Isomeren im Isomerengemisch, wird der Hauptreaktor bei einer Temperatur von 90 bis 140 °C, idealerweise 95 bis 135 °C betrieben, wie im Zusammenhang mit dem erfindungsgemäßen Verfahren näher beschrieben.

Es hat sich als besonders vorteilhaft herausgestellt, wenn das Verhältnis der Katalysatormassen vom Hauptreaktor zum Nachreaktor ist im Bereich von 1,2 bis 2 liegt, bevorzugt 1,3 zu 1,4 und idealerweise 1,35. Es hat sich überraschenderweise gezeigt, dass es hinreichend ist, die stark exotherme Reaktion beim den Reaktionsstart im Hauptreaktor durch einen intensiven Kühlkreislauf zu regeln, und lediglich die Vorlauftemperatur im Zulauf zum Nachreaktor so einzustellen, dass der moderate Temperaturanstieg von ca. 30 bis 35 °C im Nachreaktor vom Einlass zum Auslass nur noch einen limitierten und gut reproduzierbaren Einfluss auf den trans/trans-Isomerenanteil im Stoffstrom bzw. im Produkt hat, wie bereits ausgeführt.

Es kann insb. vorteilhaft sein, wenn der Hauptreaktor ein Festbettreaktor ist, der
- einen ersten Strömungsweg für das Edukt- bzw. Stoffgemisch und
- einen weiteren (geschlossenen) Strömungsweg, d.h. einem Medienumlauf für ein Wärmetauschmittel umfasst, wobei in den zweiten Strömungsweg zwei Wärmetauscher für einen indirekten Wärmeaustausch eingebunden sind:
   - einen als Kühler arbeitenden (Haupt-)Wärmetauscher und
   - einen als Heizer arbeitenden (Neben-)Wärmetauscher.

Es hat sich als sehr effektiv und vorteilhaft herausgestellt, denselben weiteren Strömungsweg, bzw. Medienumlauf zum Vorbereiten und Anfahren des Hauptreaktors mittels (Neben-)Wärmetauschers zu betreiben und die Kühlung der Hauptreaktion im produzierenden Betrieb des Hauptreaktors mittels des (Haupt-)Wärmetauschers zu betreiben.

Bei einer weiteren Ausführungsform der Anlage, kann es vorteilhaft sein, dass
die Reaktoreinheit einen weiteren Hauptreaktor als Festbettreaktor umfasst, der
   - einen ersten Strömungsweg für das Stoffgemisch und
   - einen weiteren (geschlossenen) Strömungsweg, d.h. einem.(Kühl-)Medienumlauf für ein Wärmetauschmittel umfasst, und wobei stromaufwärts zu den beiden Hauptreaktoren in der (Zu-)Leitung eine Ventileinheit vorgesehen ist, mittels welcher der Volumenstrom der Eduktmischung zw. dem ersten Hauptreaktor und dem weiteren Hauptreaktor aufteilbar, durchleitbar und/oder vollständig umschaltbar ist, und wobei beide Hauptreaktoren
   - jeweils mit einem Wärmetauscher oder
   - einem gemeinsam mit einem Wärmetauscher
für den weiteren (geschlossenen) Strömungsweg verbunden sind.

Hierbei sind die beiden in Reihe geschalteten Hauptreaktoren baugleich oder im Wesentlichen baugleich. Insbesondere sind beide Hauptreaktoren derart dimensioniert und/oder weisen entsprechende Einbauten auf, dass dieselbe bzw. die im Wesentliche gleiche Masse und/oder Volumen an Katalysator angeordnet ist oder aufnehmbar ist.

Vorteil dieser beiden Hauptreaktoren liegt darin, dass eine weitere thermische Entkopplung der ersten Reaktionsphase mit sehr starker Exothermie, der zweite Reaktionsphase mit mittlerer Exothermie und der Nachreaktion mit sehr geringer Exothermie erfolgen kann. Weiter Vorteile bestehen darin, dass bei derselben Anlagenleistung, der einzelne Hauptreaktor kleiner dimensioniert ist und somit thermisch leichter und homogener geregelt werden kann. Zeitgleich wird die Anlageleistung erhöht, weil im Wartungsfall, wie bspw. einem Katalysatorwechsel, die Anlage nicht vollständig stillgestellt werden muss. Hierzu sind die beiden Hauptreaktoren derart leitungstechnisch verschaltet, dass jeder auch alleinig vom Stoffgemisch durchströmbar ist, unter Umgehung des jeweils andere Hauptreaktors (Bypass 1).

Durch die Hydrierung im (isothermischen) Hauptreaktor mit starker Kühlung, kann die durch die Temperatur induzierte Isomerisierung stark begrenzt werden. Im (adiabatischen) Nachreaktor wird anschließend gezielt so viel Temperatur insb. durch Wärmetausch im zufließenden Stoffstrom eingeregelt und damit zugelassen, dass die Isomerisierung gerade einen spezifikationsgerechten trans/trans-Anteil im Produkt liefert. Bei einer rein isothermen Betriebsweise eines einzelnen Hauptreaktors ohne Nachreaktor, würde man zunächst zu niedrige trans/trans-Anteile und einen hohen Anteil an nicht umgesetztem MDA erhalten. Durch die Möglichkeit den Stoffstrom gezielt adiabatisch hydrieren zu lassen, kann die Temperaturentwicklung und damit die Isomerisierung im Nachreaktor vorteilhaft gesteuert werden.

Bei einer weiter verbesserten Variante ist die leitungstechnische Verschaltung derart, dass auch der Nachreaktor beim Betrieb von mindestens einem Hauptreaktor umgangen entweder kann (Bypass 2), insb. aber beim Betrieb von den beiden in Reihe geschalteten Hauptreaktoren umgangen werden kann. Bei der Verschaltungsvariante Bypass 2, übernimmt der in Strömungsrichtung als zweiter durchflossene Hauptreaktor mind. zeitweise die Funktion des Nachreaktors, so dass die Anlage ohne bzw. weitgehend ohne Leistungsabfall und/oder Änderungen der Produktqualität, insb. des jeweiligen Anteils an Isomeren im Isomerengemisch betrieben werden kann.

Bei einer weiteren Ausführungsform der Anlage, kann es vorteilhaft sein, dass in der Leitung zwischen dem mind. einen Hauptreaktor und dem zweiten Hauptreaktor mind. ein gemeinsamer Wärmetauscher angeordnet ist. Dieser gemeinsame Wärmetauscher ist in einem zentralen Ast beider Kühlmittelumläufe angeordnet. Leitungsführung und Leitungsverschaltung ist dabei derart, dass nach dem gemeinsamen Wärmetauscher das Kühlmedium zuerst in den ersten der beiden Hauptreaktoren eingeleitet wird, in dem die stärker exotherme Reaktion abläuft. Anschließend wird über eine Leitung das bereits erwärmte Kühlmedium in den stromabwärts befindlichen zweiten Hauptreaktor geleitet, so dass dieser mit einer vom ersten Hauptreaktor unterschiedlichen Vorlauftemperatur beschickt wird.

Der Vorteil besteht darin, dass es überraschenderweise beobachtet wurde, dass insb. die sehr genaue Regelung der ersten, stark exothermen Reaktionsphase für die Produktqualität entscheidend ist, so dass auf den baulichen und regelungstechnischen Aufwand eines weiteren, vollständig unabhängigen zweiten Kühlkreislaufs verzichtet werden kann. Dies insb. auch deshalb, weil über die Regelung der Vorlauftemperatur des zu den beiden Hauptreaktoren seriell nachgeschalteten Nachreaktors die vollständige Reaktion sichergestellt und geregelt werden kann, insb. der gewünschte, niedrige trans/trans-Isomerenanteil eingeregelt werden kann.

Bei einer Anlagenvariante mit einer verbesserten Regelbarkeit kann vorgesehen werden, dass in der jeweiligen (Kreuz-)Leitung der Kühlkreisläufe, mit denen der Kühlmittelauslass des ersten Hauptreaktors mit dem Kühlmitteleinlass des zweiten Hauptreaktors verbunden ist, ein bedarfsweise schaltbarer und regelbarer Wärmetauscher (Nachkühler) angeordnet ist.

Der Sumpfumlauf des Trennkessels wird bei einer Temperatur von 130 °C bis 150 °C, idealerweise 135 bis 145 °C betrieben. Der große Vorteil des baulich und regelungstechnisch sehr einfachen Trennkessels ist, dass die Siedetemperatur des Gemisches durch die Druckabsenkung ebenfalls gesenkt wird, so dass nur auf diese erniedrigte Siedetemperatur im Trennkessel aufgeheizt werden muss. Weiterhin sind bereits durch diese Maßnahme ca. 90 % des in der Eduktmischung vorliegenden Lösungsmittels, insb. THF, abtrennbar. Ein Kopfkreislauf oder Rücklaufstrom, wie bei einer Kolonne, ist hierzu nicht erforderlich. Der zur ersten Trennkolonne weitergeleitete Stoffstrom weist somit vorteilhafterweise nur eine verbleibende Lösungsmittelkonzentration von ca. 20 bis 40 Gew.% auf, idealerweise 25 bis 35 Gew.%. Somit kann die erste Trennkolonne baulich kleiner ausfallen und ist aufgrund der geringeren Masse des Stoffstroms energetisch sparsamer betreibbar.

Auf diese Weise ist die Temperatur des Stoffstrom zudem schneller absenkbar, so dass im Stoffstrom ein Anstieg des Anteils an trans/trans-Isomeren des PACM verhindert bzw. verringert wird.

Die Lösungsmittelführende (Rück-)Leitung ist der Konditionierungseinheit und/oder mind. einem geeigneten Sammeltank verbunden.

Von der Erfindung ist weiterhin eine Verfahren umfasst zur kontinuierlichen, katalytischen Hydrierung von Methylendianilin (MDA; Edukt1), insb. 4,4'-Diaminodiphenylmethan, mit einem Wasserstoffgeber (Edukt2), insb. einem gasförmigen Wasserstoffgeber, vorzugsweises Wasserstoff (H2),
wobei die Herstellung mittels einer industriellen Anlage erfolgt, **wobei** die Anlage nach mindestens einem der hierin beschriebenen Ausführungsbeispielen und Varianten ausgebildet ist, und wobei der Hauptreaktor bei einer Temperatur im Bereich von 80 °C bis 150 °C betrieben wird, und wobei im Zwischenkreislauf mittels des mind. einen Verdichters durch Dampfkompression mind. eine Temperaturerhöhung des Mediums in dem hinführenden Leitungsast von 30 °C bis 120 °C vorgenommen wird, idealerweise von 50 °C bis 90 °C.

Bei einer weiteren Ausführungsform des Verfahrens kann ein weiterer Vorteil darin bestehen, dass der mind. eine Hauptreaktor bei einem Druck im Bereich von 60 bar bis 120 bar betrieben wird, idealerweise im Bereich von 70 bis 110 bar. Bei einer weiteren vorteilhaften Verfahrensführung kann vorgesehen werden, dass der Druck im Hauptreaktor 70 bis 100 bar beträgt, idealerweise 80 bis 90 bar beträgt. Besonders bevorzugt ist ein Druck von ca. 85 bis 90 bar.

Bei einer bevorzugten Ausführungsform des Verfahrens kann vorgesehen werden, dass eine kontinuierliche, katalytische Produktion von Methylenbis(cyclohexylamin) erfolgt, insb. die Produktion von 4,4`-Diaminodicyclohexylmethan (PACM), bevorzugt 4,4'-Diaminodicyclohexylmethan (PACM) mit niedrigen Anteilen an trans/trans-Isomeren. Bei einer bevorzugten Verfahrensvariante erfolgt eine kontinuierliche, katalytische Herstellung von Methylenbis(cyclohexylamin), insb. eine Produktion von PACM, nach der Formel (I)

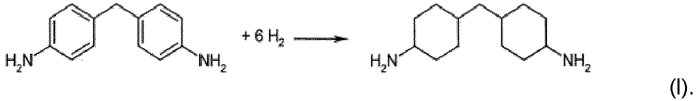

Bei einer weiteren Ausführungsform des Verfahrens kann ein weiterer Vorteil darin bestehen, dass vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, die Betriebstemperatur des Hauptreaktors erhöht und Temperatur im Stoffstrom im Einlass (Feed) des Nachreaktors gleichgehalten oder gesenkt wird, wobei die Temperaturerhöhung oder -Absenkung linear und/oder schrittweise erfolgt.

Mit der genannten Anlagenvariante ist somit der folgende Verfahrensverlauf zu einer Erreichung eines trans/trans-Anteils im PACM von 17 bis 25 Gew.% über der Zeit möglich.

Auch wenn der Hauptreaktor vorliegend als "isotherm" bezeichnet wird, wird dieser ideale Zustand bei industrieller Anwendung nur bedingt erreicht, so dass innerhalb des Hauptreaktors wegen unvollständigem Wärmeabtransport, sich ein Temperaturgradient von ca. 5 bis 10 °C in radiale und auch in Strömungsrichtung ausbildet.

Bei einer weiteren Ausführungsform des Verfahrens kann ein weiterer Vorteil darin bestehen, dass im Verteilerkreislauf eine mehrstufige Druckerhöhung in dem hinführenden Leitungsast erfolgt, wobei nach dem zweiten Verdampfer und vor dem ersten Verdichter ein Eingangsdruck von 1,5 bar bis 5 bar und eine Temperatur von 100°C bis 150°C vorliegt.

Bei einer weiteren Ausführungsform des Verfahrens kann ein weiterer Vorteil darin bestehen, dass im Verteilerkreislauf im ersten Leitungsast eine mehrstufige Druckerhöhung erfolgt, wobei nach dem letzten Verdichter und vor dem ersten als Wärmesenke wirkenden Wärmetauscher ein Druck von 3 bar bis 30 bar und eine Temperatur von 130°C bis 300°C vorliegt.

Bei einer weiteren Ausführungsform des Verfahrens kann ein weiterer Vorteil darin bestehen, dass die vom Sammelkreislauf in den ersten Verdampfer eingebrachte Energie mittels des Zwischenkreislaufs und dem mind. einen eingebundenen Verdichter sowie dem ggf. kreuzweise eingebundenen Wärmetauscher
- um mind. Faktor 1,1 bis 2,5 und/oder
- die Ausgangstemperatur des ersten Verdampfers
   um mind. den Faktor 1,2 bis 3,0
angehoben wird.

Bei einer weiteren Ausführungsform des Verfahrens kann ein weiterer Vorteil darin bestehen, dass das MDA (Edukt1) eine Mischung der folgenden Monomere umfasst: 4,4'-MDA, 2,4'-MDA und 2,2'-MDA, wobei der Anteil an 4,4'-MDA vorteilhafterweise im Bereich von 75 bis 98 mol-% beträgt, idealerweise 85 bis 95 mol-%, bevorzugt 90 mol-%. Der Anteil an 2,4` MDA im Eduktgemisch beträgt vorteilhafterweise 7 bis 15 mol-%, bevorzugt 8 bis 12 mol-%, idealerweise 9 bis 10 mol-%. Der Anteil an 2,4` MDA im Eduktgemisch beträgt vorteilhafterweise 7 bis 15 mol-%, bevorzugt 8 bis 12 mol-%, idealerweise 9 bis 10 mol-%.

Idealerweise ist der Isomerenanteil an trans/trans- PACM im Produkt im Bereich von 15 bis 30 Gew.%, idealerweise 16 bis 25 Gew.%

Insgesamt ist es vorteilhaft, wenn der Reaktionsschritt im Hauptreaktor hierbei isotherm oder weitgehend isotherm und der Reaktionsschritt im Nachreaktor adiabat oder weitgehend adiabat betrieben wird. Zum Zeitpunkt t₀, dem Verfahrensstart kann neben dem Hauptreaktor auch der Katalysator im Nachreaktor erneuert oder regeneriert vorliegen. Vorteilhafterweise wird der Zyklus zur Regeneration oder Erneuerung des Nachreaktors eigenständig und vom Hauptreaktor unabhängig bestimmt, insb. wenn o.g. Zusammenwirken der Reaktionsanteile in beiden Reaktoren nicht mehr die Produktion des gewünschten niedrigen trans/trans-Anteils im PACM ermöglicht.

Bei einer vorteilhaften Ausführungsform des Verfahrens kann vorgesehen sein, dass die Temperatur des Eduktstroms am Einlass des Hauptreaktors 90 bis 140°C beträgt, idealerweise 100 bis 135 °C.

Bei einer weiteren vorteilhaften Ausführungsform des Verfahrens kann vorgesehen sein, dass - die Temperatur am Eingang des Hauptreaktors im Wesentlichen der Temperatur am Eingang des Nachreaktors entspricht, wobei im Wesentlichen einen Bereich oder Unterschied von +/- 10 °C meint und/oder der Druck am Eingang des Hauptreaktors im Wesentlichen dem Druck am Eingang des Nachreaktors entspricht, wobei im Wesentlichen einen Bereich oder Unterschied von +/- 5 bar meint.

Insgesamt sollen alle Aspekte, Vorteile und Ausführungen zur Anlagen oder in Zusammenhang mit deren Beschreibung genannt sind, identisch oder in analoger Weise auch für das Verfahren gelten und umgekehrt, sofern nicht etwas Abweichendes dargelegt ist und/oder eine technische Unmöglichkeit der analogen Anwendung gegeben ist.

Nachfolgend wird die erfindungsgemäße Lösung anhand von Ausführungsbeispielen im Detail beschrieben. Es zeigen:
- Fig. 1: eine Anlage als Prozessfließbild
- Fig. 2: eine erste Ausführungsform einer zweistufigen Entspannung
- Fig. 3: eine weiter Ausführungsform einer zweistufigen Entspannung
- Fig. 4: eine Variante der Ausführungsform nach Figur 3,
- Fig. 5: eine Variante der Ausführungsform nach Figur 3,
- Fig. 6: eine Variante der Ausführungsform nach Figur 3,
- Fig. 7: eine weitere Ausführungsform der Reaktoreinheit und
- Fig. 8: eine weiter Ausführungsform der Reaktoreinheit.

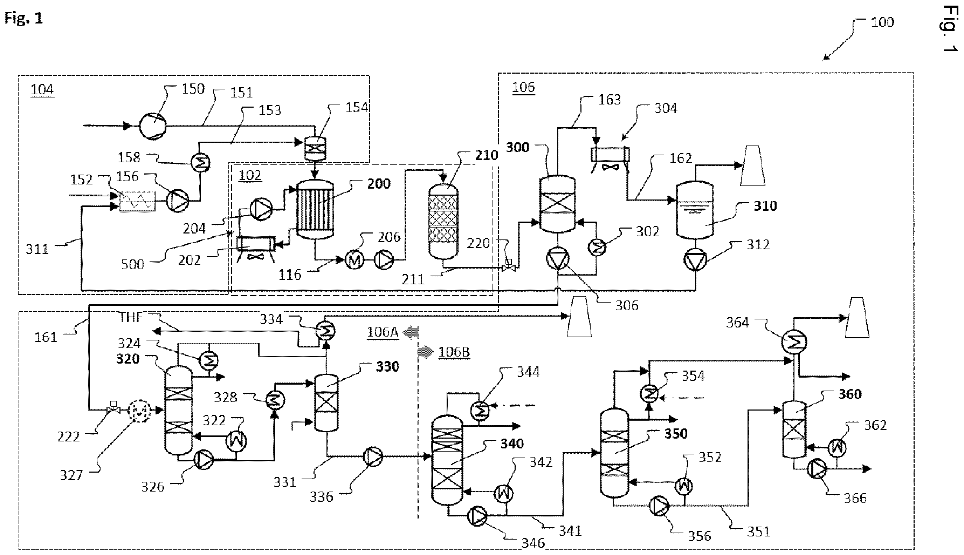

Die Figur 1 zeigt die Anlage 100 zur kontinuierlichen Herstellung von 4,4'-Diaminodicyclohexylmethan (PACM) durch Hydrierung von Methylendianilin (MDA; Edukt1), insb. 4,4'-Diaminodiphenylmethan, mit einem Wasserstoffgeber (Edukt2), wobei der Wasserstoffgeber als gasförmiger Wasserstoff (H2) zugeführt wird. Die Anlage 100 umfasst eine Konditionierungseinheit 104 für die Edukte, eine Reaktoreinheit 102 und eine Trenneinheit 106.

Die Konditionierungseinheit 104 ist gestrichelt umrahmt und umfasst (Zu-)Leitungen für das Edukt1 und den Wasserstoff (Edukt2) sowie das Lösungsmittel. Weiterhin umfasst die Konditionierungseinheit eine Verdichtereinheit 150, nachfolgend Verdichter genannt in der den Wasserstoff zuleitenden Leitung 151, einen Mischer 152 in der das Lösungsmittel und das Edukt1 zuführenden Leitung. Zudem sind in der die Mischung aus Edukt1 und Lösungsmittel zuführenden Leitung 153 eine Pumpe 156 und ein Wärmetauscher 158 angeordnet. Die Leitungen 151, 152 münden in einen Mischbehälter 154, der stromaufwärts zum Hauptreaktor 200 angeordnet ist. Der Mischbehälter 154 dient der intensiven Vermischung der Edukte und dessen Auslauf bildet den Zulauf für den Hauptreaktor 200.

Die Reaktoreinheit 102 ist gestrichelt umrahmt und umfasst im Wesentlichen den Hauptreaktor 200, einen Kühlumlauf 500, eine Nachreaktor 210 und einen Wärmetauscher 206 in der Zulaufleitung zum Nachreaktor 210. In den Kühlumlauf 500 ist eine Pumpe 204 und ein Wärmetauscher 202 eingebunden, wobei das umlaufende Kühlmittel im Hauptreaktor 200 die mit Katalysatormaterial befüllten Trägerelemente umströmt. Im gezeigten Beispiel werden die mit Katalysatormaterial befüllten Trägerelemente im Gleichstromrichtung angeströmt. Der Nachreaktor 210 ist über die Leitung 211 mit dem Trenneinheit 106, bzw. deren ersten Trennstufe verbunden.

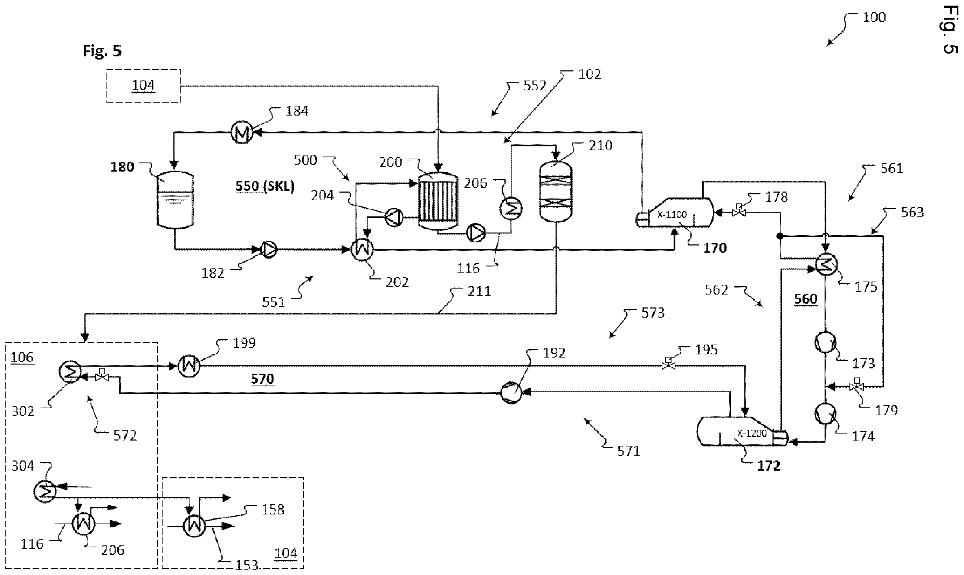

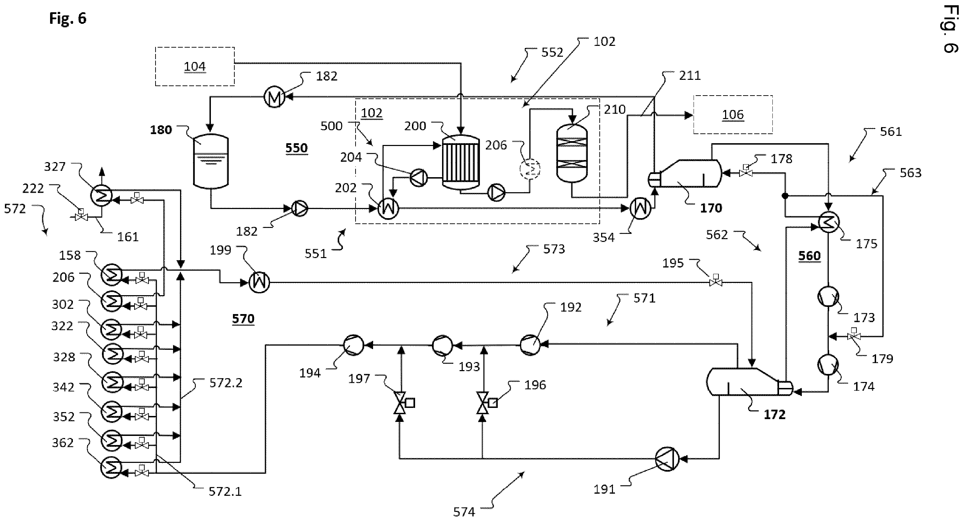

Der Wärmetauscher 202 im Kühlumlauf 500 ist als luftgekühlter Wärmetauscher 202 dargestellt, kann aber auch alternativ ausgebildet sein, um bspw. mittels eines fließenden Kühlmediums, wie bspw. ein Öl, Wasser oder eine Sole, um im indirekten Wärmeaustausch das Kühlmedium des Kühlumlaufs 500 zu temperieren. In den Kühlumlauf 500 ist weiterhin bei einer nichtdargestellten Anlagenvariante ein Wärmetauscher eingebunden, analog den Wärmetauschern 208, 209 der Figuren 5, 6. Dieser wird mit einem Heizmedium betrieben und dient im Anfahrschritt des Hauptreaktors 200 zur Temperierung des Hauptreaktors 200 auf ca.. In dem gezeigten Anlagen- und Verfahrensbeispiel, bei dem Ziel ein möglichst niedriges trans/trans-Isomerenverhältnis von ca. 17 bis 23 Gew.% zur realisieren, wird der mit frischem oder regeneriertem Katalysator befüllte Hauptreaktor 200 auf eine Temperatur von ca. 90 °C mittels des Wärmetauschers 208 vorgeheizt. Der Hauptreaktor 200 wird bei einem Druck von 87 bis 88 bar betrieben.

Die Trenneinheit 106 ist gestrichelt umrahmt und umfasst eine Mehrzahl von Trennapparaten zur Abtrennung des Lösungsmittel insb. in einer ersten Trennstufe und des Produkts PACM, insb. in einer zweiten Trennstufe, von restlichem Edukt und Nebenprodukten. Die erste Trennstufe (nicht ausgewiesen) umfasst einen Trennkessel 300 (Flashbehälter) an den eine Sumpfumlauf angeschlossen ist, in den ein Wärmetauscher 302 und eine Pumpe 306 eingebunden ist. Der Kopfauslass des Trennkessels 300 ist mit einem Wärmetauscher 304, einem Kondensator, verbunden, zu dem stromabwärts ein Sammelbehälter 310 für das Lösungsmittel vorgesehen ist. Mittels des Wärmetauschers 304 (Kondensator) werden ca. 80% des Lösungsmittels, vorliegend THF, auskondensiert und könnten gesammelt bzw. zurückgeleitet werden. In der Flash-Stufe ist ein Energiebedarf für den Wärmetauscher 302 im Sumpfumlauf des Trennkessels 300 von ca. 1400 kW erforderlich.

Der Kondensator 304 ist als Wärmetauscher in der Bauform eines luftgekühlten Apparates dargestellt, kann aber auch alternativ ausgebildet sein, um bspw. mittels eines fließenden Kühlmediums, wie bspw. Kühlwasser oder einem zur Wärmeintegration geeigneten Medium, um im indirekten Wärmeaustausch den eingangs dampfförmigen Stoffstrom mind. teilweise zu kondensieren und zu kühlen.

Die hierin genannten Energiemengen sind kalkuliert für eine Anlagenleistung des Produktes PACM bei der genannten Synthesereaktion von ca. 3,37 t/h, sowie eine Nebenproduktleistung von ca. 0,4 t/h an HB und ca. 0,05 t/h an LB. Das Verhältnis der Massenströme von THF zu MDA betrugt 4,2 bis 4,5.

Weiterhin umfasst die erste Trennstufe der Trenneinheit 106 zur weitergehenden Abtrennung des Lösungsmittels einen erste Trennkolonne 320 und eine zweite Trennkolonne 330, wobei die zweite Trennkolonne 330 als Stripping Kolonne ausgebildet ist. Hierzu wird vorteilhafterweise Stickstoff (N2) als Stripping-Medium eingesetzt, das im Gegenstrom zum Stoffstrom durch die Kolonne geleitet wird. Mittels der im Sumpfauslass des Trennkessels 300 befindlichen Pumpe 306 ist der an Lösungsmittel verarmte Stoffstrom über die Leitung 161 zur ersten Trennkolonne 320 ableitbar. In der Leitung 161 ist eine Entspannungseinheit 222 vorgesehen, die im gezeigten Beispiel als regelbares Ventil ausgebildet ist. Der ersten Trennkolonne 320 wird der Stoffstrom über einen zentralen Zulauf wie dargestellt eingeleitet. Weiterhin ist stromaufwärts zur ersten Trennkolonne 320 ein (Vorlauf-)Wärmetauscher 327 (gestrichelt dargestellt) angeordnet, der eine Option zur Beheizung der ersten Trennkolonne 320 darstellt.

In der ersten Trennkolonne 320 wird die Lösungsmittelkonzentration von 30 Gew.% im Zulauf über den produktreichen Stoffstrom der Leitung 161 auf ca. 2 Gew.% an restlichem Lösungsmittel verringert.

Diese erste, mit (Struktur-)Packungen bestückte, Trennkolonne 320 ist an einen Sumpfumlauf angeschlossen, in den ein Wärmetauscher 322 und eine Pumpe 326 eingebunden sind. Weiterhin ist am Kolonnenkopf der ersten Trennkolonne 320 ein Kopfumlauf angeordnet, in den ein Wärmetauscher 324 eingebunden ist, der als Kondensator ausgebildet ist. Aus dem Kopfumlauf führen zwei Ableitungen für den lösungsmittelreichen Stoffstrom, wobei eine der beiden Ableitungen in die Kopfableitung der stromabwärts befindlichen Trennkolonne 330 (Stripping-Kolonne) einmündet.

Insbesondere ist das in der ersten Trennstufe abgetrennte Lösungsmittel über die Leitung 311 in einen Sammeltank (nicht dargestellt) und/oder den Mischer 152 der Konditionierungseinheit 104 leitbar. In der vom Nachreaktor 210 zum Trennkessel 300 führenden Leitung 211 ist eine Entspannungseinheit 220 vorgehen, die im vorliegenden Beispiel als steuerbares Ventil ausgebildet ist.

Über die Leitung 321 wird aus dem Sumpfauslass der ersten Trennkolonne 320 der produktreiche Stoffstrom mit einem Restanteil von ca. 2 Gew.% Lösungsmittel (THF) in den Kopf der zweiten Kolonne 330, der Stripping-Kolonne, geleitet. In dieser Leitung 321 ist ein Wärmetauscher 328 eingebunden. Die zweite Kolonne 330 weist mind. eine innere Packung auf, wobei unterhalb der Packung eine Gaszuleitung für insb. ein Inertgas (Strippinggas) angeordnet ist, so dass der eingeleitete produktreiche Stoffstrom im Gegenstromprinzip zum Strippinggas die zweite Kolonne 330 durchströmt und von Lösungsmittel weiter abgereichert wird. In dem gezeigten Anlagenbeispiel wird Stickstoff (N2) als Strippinggas eingesetzt. Der lösungsmittelreiche Dampf wird über den Kopfauslass in einen Kondensator 334 geleitet und gemeinsam mit dem von Kopfauslass der ersten Trennkolonne 320 kommenden und die Kopfableitung der zweiten Trennkolonne 330 eingeleitete Dampfstrom dem Kondensator 334 zugeleitet. Der im Wärmetauscher 334 auskondensierte Lösungsmittelstrom wird zur Konditionierungseinheit 104 zurückgeleitet, wobei der nicht-kondensierbare Anteil aus dem Wärmetauscher 334 abgeleitet und bspw. vollständig thermisch oxidiert wird.

Vom Sumpfauslass der zweiten Trennkolonne 330 wird der produktreiche Stoffstrom über die Leitung 331, in die die Pumpe 336 eingebunden ist, der dritten Trennkolonne 340 zugeführt. Die zweite Trennstufe 106B der Trenneinheit 106 dient insb. der Abtrennung der Nebenprodukte LB und/oder HB vom Produkt PACM.

Die zweite Trennstufe umfasst im Wesentlichen drei Trennkolonnen, wobei der dritten Trennkolonne 340, die erste der zweiten Trennstufe, der Stoffstrom zentral zugeführt wird. Die dritte Trennkolonne ist mit einem Sumpfumlauf verbunden, in den ein Wärmetauscher 342 und eine Pumpe 346 eingebunden sind. Der produktreiche Stoffstrom wird über die Leitung 341 aus dem Sumpfablauf zur vierten Trennkolonne 350 geleitet. Weiterhin ist die dritte Trennkolonne 340 mit einem Kopfumlauf verbunden, in den ein Wärmetauscher 344 eingebunden ist. Aus diesem Kopfumlauf wird auskondensiertes LB als erstes Nebenprodukt abgeleitet.

Der produktreiche Stoffstrom wird über die Leitung 341 der vierten Trennkolonne 350 zentral zugeführt. Die vierte Trennkolonne 350 ist mit einem Sumpfumlauf verbunden, in den ein Wärmetauscher 352 und eine Pumpe 356 eingebunden sind. Weiterhin ist die vierte Trennkolonne 350 mit einem Kopfumlauf verbunden, in den ein als Kondensator ausgebildeter Wärmetauscher 354 eingebunden ist. Der produktreiche Stoffstrom wird über die Kopfableitung aus dem Kopfumlauf als Kondensat ausgeleitet. Vorliegend wird gemäß dem gezeigten Beispiel, der nichtkondensierte Dampf- und/oder Gasstrom in die Kopfausleitung der stromabwärtsbefindlichen fünften Trennkolonne 360 eingeleitet. Nachfolgend wird über einen weiteren Wärmetauscher 364 (Kondensator) Produkt weiter auskondensiert und ausgeleitet. Der produktarme Stoffstrom wird über die Sumpfableitung der vierten Trennkolonne 350 über die Leitung 351 abgeleitet und der fünften Trennkolonne 360 in deren Kopfteil eingeleitet. Dieser Stoffstrom ist stark angereichert an HB, einem zweiten Nebenprodukt. Die fünfte Trennkolonne 360 ist mit einem Sumpfumlauf verbunden, in den ein Wärmetauscher 362 und eine Pumpe 366 eingebunden sind. Weiterhin weist die Trennkolonne 360 einen Kopfauslass auf, der zu dem vorgenannten als Kondensator ausgebildeten Wärmetauscher 364 führt. In diesem Kondensator 364 wird ein weiterer produktreicher Stoffstrom als Kondensat auskondensiert und ausgeleitet, wobei der nicht-kondensierbare Anteil gasförmig ausgeleitet wird. Dieser kann nachfolgend insb. vollständig oxidiert werden.

Die Reaktoreinheit 102 wird bzgl. des Stoffstroms auf einem ersten, hohen Druckniveau von ca. 60 bis 120 bar betrieben, die erste Trennstufe 106A der Trenneinheit 106 wird bei einem zweiten, niedrigen Druckniveau von 4 bis 12 bar betrieben und die erste Trennkolonne 320 und die zweite Trennkolonne 330 werden auf einem dritten, geringfügig erhöhten Druckniveau von 1,05 bis 2,5 bar betrieben. In dem gezeigten Beispiel ist das erste Druckniveau 80 bis 90 bar, das zweite Druckniveau 4,5 bis 7,5 bar und das dritte Druckniveau 1,1 bis 1,2 bar.

Mit der direkten Temperaturregelung des Hauptreaktors 200 über den Kühlkreislauf 500 in Reihe mit dem ungekühlten Nachreaktor 210, hat sich überraschenderweise ein gegenüber dem Stand der Technik verringerter Energiebedarf und eine vereinfachte, stabilere Verfahrensführung gezeigt. Ohne auf eine spezielle Interpretation gebunden zu sein, wird dieser Erfolg darin gesehen, dass der Kühlkreislauf 500 nur gezielt für die sehr heftige Anfangsreaktion zur Ableitung der exothermen Reaktionswärme ausgelegt werden muss, wobei die noch bedeutsame Nachreaktion alleinig über die Zulauftemperatur mittels des stromaufwärts befindlichen Wärmetauscher 206 eingestellt werden muss. Der Stoffstrom wird durch die bereits stark abgeschwächte Reaktion im Nachreaktor 210 nur noch um ca. 5 bis 15 °C erwärmt und kann in diesem Niveau unproblematisch in den Trennkessel 300 entlassen werden.

Insgesamt sind in den Figuren in den Apparaten, wie Reaktoren, Trennkolonnen, Behältern etc., über die entsprechenden Symbole Einbauten, wie Packungen, Trennebenen, Trägerelemente etc. skizziert. Diese deuten jeweils vorteilhafte Ausführungsformen an, betreffend die Anzahl, Art und/oder relative Lage zur jeweils zu- oder abführenden Leitung. So ist bspw. mit der Darstellung ersten Trennkolonne 320 angedeutet, dass vorteilhafterweise der (Feed-)Stoffstrom über die Leitung 161 derart eingeleitet wird, dass zwischen dem Kopfumlauf und dem Sumpfumlauf jeweils mind. eine (theoretische) Trennebene vorhanden ist. Die Ermittlung der konkreten Art und/oder Anzahl von Trennebenen ist dem Fachmann bekannt und kann in geeigneter Weise variiert bzw. vorgesehen werden.

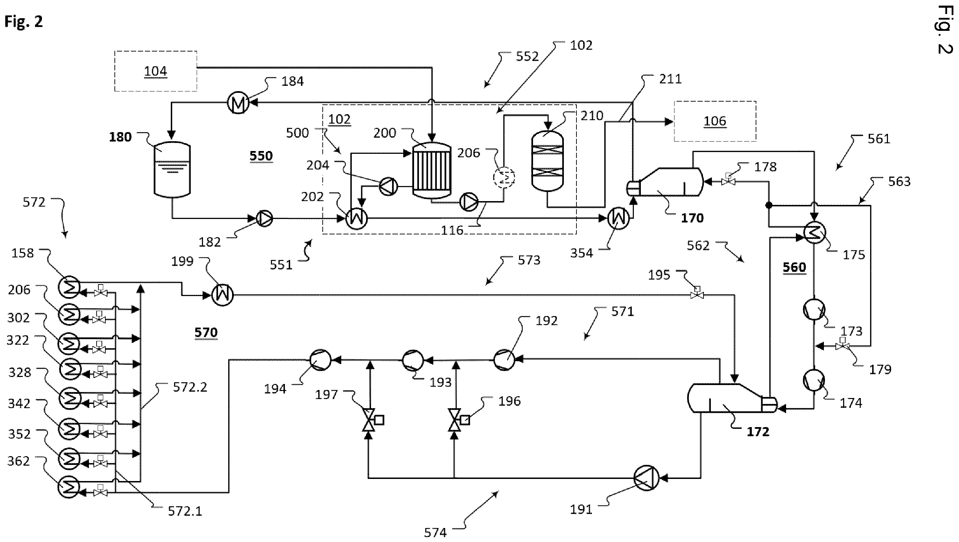

Figur 2 zeigt eine erste Ausführungsform einer Energieverschaltung, wobei drei Kreisläufe vorgesehen sind, nämlich ein Sammelkreislauf 550, eine Zwischenkreislauf 560 und ein Verteilkreislauf 570. Der Sammelkreislauf 550 einen Sammeltank 180, eine erste Leitung 551, einen Verdampfer 170 und eine zweite Leitung 552, wobei die erste Leitung 551 den Sammeltank 180 mit einem Einlass eines Wärmetauscher-Teils (WT-Teil) des Verdampfers 170 verbindet und die zweite Leitung 552 einen Auslass des WT-Teils des Verdampfers 170 mit dem Sammeltank 180 verbindet. Es ist somit nur der WT-Teil des Verdampfers 170 Teil des Sammelkreislaufs 550.

In die erste Leitung 551 sind der Wärmetauscher 202 des Kühlmittelumlaufs 500 des Hauptreaktors 200 und der Wärmetauscher 354 aus dem Kopfumlauf der vierten Trennkolonne 350 als Wärmequellen eingebunden, sowie eine Pumpe 182. In der zweiten Leitung 552 ist in dem gezeigten Beispiel ist im Vorlauf zum Sammeltank 180 ein Wärmetauscher 182 eingebunden, der im Wesentlichen dazu dient, bedarfsweise und aus Regelungsaspekten die erforderliche Zulauftemperatur (Kühlung) des Sammeltanks 180 und/oder die Temperatur in der ersten Leitung 551 einzustellen, um den Temperaturgradienten für die erforderliche Kühlung der eingebundenen Wärmetauscher 202, 354 zu gewährleisten. Die erste Leitung 551 kann auch als Sammel- oder Feedleitung betrachtet werden und die zweite Leitung 552 als Rückleitung. Als im Kreislauf strömendes Medium ist in dem gezeigten Beispiel Wasser vorgesehen, wobei auch eine Sole oder ein Öl genutzt werden könnte.

Durch den Sammelkreislauf werden auf vorteilhafter Weise mehrere Wärmequellen "eingesammelt" und derart gesammelt die Energiemenge des im Kreislauf geführten Mediums, hier insb. Wasser, an den ersten Verdampfer zu übertragen. Dies verringert den konstruktiven Aufwand erheblich, verglichen mit einer direkten Verschaltung der betroffenen Wärmetauscher.

Der Zwischenkreis 560 umfasst den vorstehend genannten Verdampfer 170, eine erste Leitung 561, zwei Verdichter 173, 174, einen (Vorlauf-)Wärmetauscher 175, einen weiteren Verdampfer 172, einen weitere Leitung 562 und eine Entspannungseinheit 178. Von dem ersten Verdampfer 170 ist der Kesselteil (K-Teil) Teil des Zwischenkreislaufes 560 und von dem zweiten Verdampfer 172 ist der WT-Teit eingebunden, d.h. vom Wärmetauschmedium des Zwischenkreislaufes 560 durchflossen. Der (Vorlauf-)Wärmetauscher 175 ist derart verschaltet, dass dieser in der zweiten Leitung 562 stromabwärts zum zweiten Verdampfer 172 und im Vorlauf mind. einer der Verdichter 173, 174 in der ersten Leitung 561 eingebunden ist. Weiterhin ist eine (Brücken-)Leitung 563 vorgesehen, über die abzweigend von der zweiten Leitung 562 das Wärmetauschmedium in die erste Leitung 561 einleitbar ist. Der Leitungsknoten für die Abzweigung der (Brücken-)Leitung 563 ist stromabwärts zum Wärmetauscher 175 in der zweiten Leitung 562 und die Einleitung in die erste, hinführende Leitung erfolgt zwischen die beiden Verdichter 173, 174. Als Wärmetauschmedium dient vorteilhafterweise ein im Vergleich zum Wasser leiterflüchtiges Medium, wie insb. ein Alkohol mit 1 bis 6 Kohlenstoffatomen, insb. Methanol, 2-Propanol, Butanol oder n-Butanol. Stromaufwärts zum Einlass der zweiten Leitung 562 in den K-Teil des ersten Verdampfers 170 ist die Entspannungseinheit 178 angeordnet.

In der ersten, hinführenden Leitung wird das verdampfte Medium, hier Methanol bei einem Druck von ca. 2,6 bar geführt und in zwei Kompressionsstufen überhitzt. Der Druck wird durch die Verdichter 172, 174 auf ca. 9,5 bar angehoben, wodurch der Medienstrom überhitzt wird und eine Temperatur von ca. 177 °C nach dem zweiten Verdichter 174 aufweist. Der Medienstrom wird im WT-Teil des zweiten Verdampfers 172 abgekühlt, so dass das Medium auskondensiert und als flüssige Phase vorliegt.

Neben der Möglichkeit das Energieniveau bedeutsam anzuheben, besteht ein weiterer Vorteil des Zwischenkreislaufs darin, dass das Wärmetauschmedium selbst einen zusätzlicher Freiheitsgrad darstellt. So kann das Arbeitsmedium so gewählt werden, dass es optimal für die prozessbedingten Temperaturen des Sammelkreislaufs und des Versorgungskreislaufes optimal geeignet ist und zu minimalen Kompressionskosten für den Betrieb der Verdichter führt.

An den zweiten Verdampfer 172, dessen WT-Teil in den Zwischenkreislauf 560 eingebunden ist, schließt sich der Verteilerkreislauf 570 an, in den der K-Teil dieses Verdampfers 172 eingebunden ist. Der Verteilerkreislauf 570 umfasst einen ersten, hinführenden Leitungsast 571, einen zweiten, verteilenden Leitungsast oder Leitungsabschnitt 572 und einen dritten, rückführenden Leitungsast 573, auch (Rück-)Leitung genannt. Ein Kopfauslass des Verdampfers 172 führt in den ersten Leitungsast 571, wobei im gezeigten Ausführungsbeispiel drei in Reihe geschaltete Verdichter 192, 193, 194 in den ersten Leitungsast 571 eingebunden sind. Weiterhin umfasst der Verteilerkreislauf 570 eine (Sumpf-)Leitung 574, die von einem Sumpfauslass des Verdampfers 172 über zwei Leitungsknoten den ersten Leitungsast 571 mündet, wobei eine Pumpe 191 in der (Sumpf-)Leitung 574 eingebunden ist. Der erste Einleitung von Heizmedium erfolgt über den ersten Leitungsknoten zwischen dem ersten Verdichter 192 und dem zweiten Verdichter 193, die zweite Einleitung von Heizmedium erfolgt zwischen dem zweiten Verdichter 193 und dem dritten Verdichter 194. Stromaufwärts zu den jeweiligen, nicht bezeichneten Leitungsknoten ist jeweils ein regelbares Ventil 196, 197 angeordnet.

In dem verteilenden Leitungsabschnitt 572 sind als Wärmesenken zu versorgenden Wärmetauscher die zueinander parallel geschalteten Wärmetauscher 158, 206, 302, 322, 328, 342, 352 und 362 eingebunden. Eine zentrale Verteilleitung 572.1 führt zu den Wärmetauschern und eine zentrale Sammelleitung 572.2 wird von allen Wärmetauschern gespeist und führt in die (Rück-)Leitung 573. Der verteilende Leitungsabschnitt 572 ist über die (Rück-)Leitung 573 mit dem K-Teil des zweiten Verdampfers 172 verbunden. In diese (Rück-)Leitung 573 sind ein Wärmetauscher 199 und eine Druckregeleinheit 195 eingebunden, worüber der Enddruck des dritten Verdichters 194 von 20 bar wieder auf das Niveau des K-Teils des zweiten Verdampfer von ca. 2.5 bis 3 bar entspannt wird. Die (Rück-)Leitung 573 ist mit einem Einlass des K-Teils des Verdampfers 172 verbunden.

Die beiden Verdampfer 170, 172 sind so genannte Kettle-Type-Verdampfer, die einen für ein erstes strömendes Medium geschlossenen Wärmetauscher-Teil (WT-Teil) und einen für ein weiteres strömendes Medium offenen Kessel-Teil (K-Teil) aufweisen. Die WT-Teile weisen jeweils einen Einlass und einen Auslass auf, wobei das Wärmetauschmedium in geschlossenen Kanälen oder Rohren geführt wird, wie bspw. mind. einem Rohrbündel. Die K-Teile weisen jeweils mind. einen Einlass und jeweils einen (Kopf- oder Dampf-)Auslass auf, wobei der zweite Verdampfer 172 auch einen (Sumpf-)Auslass aufweist. Über insb. den mind. einen (Sumpf-)Einlass eingeleitetes Medium wird mittels des jeweiligen WT-Teils bzw. des zugehörigen Wärmetauschers erwärmt und mind. teilweise verdampft. Das jeweilige K-Teil kann zweiteilig sein oder zwei Teilräume aufweisen. Hierbei ist eine zentraler K-Teil vorhanden, in welchen auch der Wärmetauscher des WT-Teils hineinragt und der Energieeintrag in den K-Teil erfolgt. Der weitere Teilraum ist in einem seitlichen oder äußeren K-Teil angeordnet. Dieser kann vorteilhafterweise, aber nicht zwingend, durch Einbauten als eine für das flüssige Medium beruhigte Zone aus gebildet sein und/oder ist dadurch bestimmt, dass der Wärmetauscher des WT-Teil in diesen Teilraum nicht hineinragt.

Der wasserführende Verteilerkreislauf weist am hinführenden Leitungsast unmittelbar stromabwärts zum zweiten Verdampfer 172 eine Temperatur von 130 °C und 2,7 bar auf. Nach dem ersten Verdichter 192 beträgt der Druck 5,4 bar, der mit einem Energieverbrauch von 238 kW erreicht wird, nach dem zweiten Verdichter 192 beträgt der Druck 10,8 bar, der mit einem elektrischen Energieverbrauch von 295 kW erreicht wird und nach dem dritten Verdichter 193 beträgt der Druck 20 bar bei einer Temperatur von 250 °C, erreicht durch weiteren elektrischen Energieverbrauch von 250 kW für den dritten Verdichter 193.

Zur Versorgung der in der Figur 2 gezeigten Wärmetauscher, die insb. die Sumpfwärmetauscher der Trennkolonnen sind, muss ein sehr hohes Temperaturniveau bereitgestellt werden, so dass über den Bedarfs- und Regelungswärmetauscher 199 das zirkulierende Medium wieder gekühlt werden muss. Die erforderliche Kühlleistung beträgt im gezeigten Beispiel ca. 368 kW, die in der (Rück-)Leitung 573 vor der Druckregelungseinheit 195 bzw. vor Eintritt in den zweiten Verdampfer 172 bereitgestellt werden muss.

Ein bedeutsamer Vorteil des Verteilerkreislaufes kann darin gesehen werden, dass ein zentraler Dampfkreislauf geschaffen wurde und somit Dampf zentral erzeugt und auf alle als Verbraucher (Wärmesenke) arbeitenden Wärmetauscher verteilt werden kann, anstelle der direkten Verschaltung von Wärmequellen und Wärmesenken. Weiterhin kann der Verteilerkreislauf als zentraler Dampfkreislauf im Bedarfsfall (z.B. beim Anfahren der Anlage) mindestens zeitweise mittels einer alternativen Wärme- oder Dampfquelle gespeist werden.

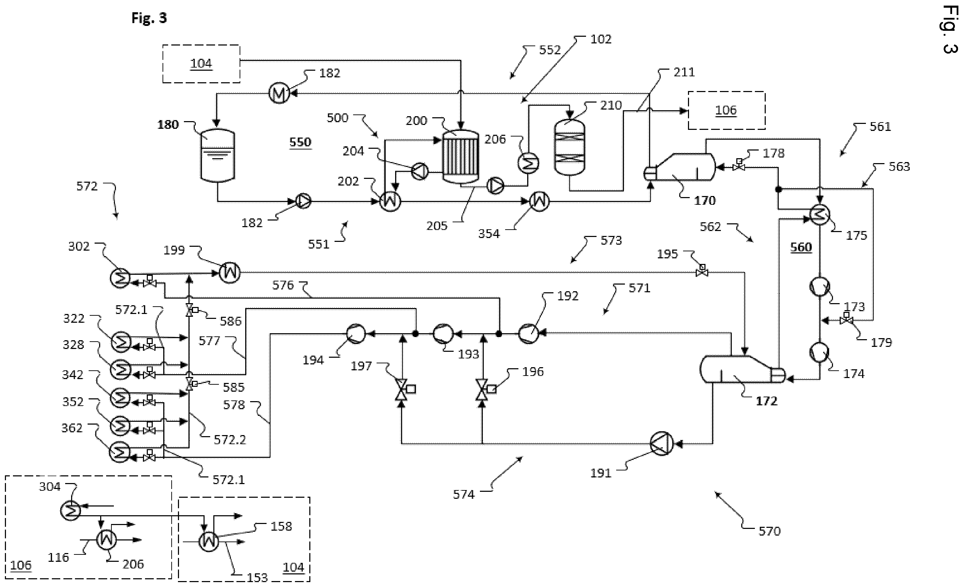

Figur 3 zeigt eine Ausführungsform, in der der Sammelkreislauf 550 und der Zwischenkreislauf 560 analog ausgebildet sind zur Figur 2. Der Verteilerkreislauf 570 unterscheidet sich zur Ausführungsform nach Figur 2 dadurch, dass der Leitungsabschnitt 572, über den die Energieverteilung an die als Wärmesenken arbeitende Wärmetauscher erfolgt, in drei Teilabschnitte aufgeteilt ist, wobei die drei Teilabschnitte zueinander parallel geschaltet sind:
- ein Hochdruck-Teilabschnitt (HD-Teilabschnitt), angeschlossen stromabwärts zum dritten Verdichter 194,
- ein Mitteldruck-Teilabschnitt (MD-Teilabschnitt), angeschlossen stromabwärts zum zweiten Verdichter 193 und
- ein Niederdruck-Teilabschnitt (ND-Teilabschnitt), angeschlossen stromabwärts zum ersten Verdichter 192.

Hierbei meint "ND-Teilabschnitt/Kreislauf", dass in diesem Teilabschnitt oder Kreislauf "weniger Verdichter und/oder weniger Verdichterleistung" eingebunden sind, als in den HD-Teilabschnitt/Kreislauf", dass der mögliche Enddruck und damit auch die Endtemperatur (ohne weiteren Wärmetausch) aufgrund der Anzahl und/oder der Bauart der Verdichter im ND-Teilabschnitt/Kreislauf" niedriger ist als in dem HD-Teilabschnitt/Kreislauf. Anders ausgedrückt, ein niedrigerer Druck des erzeugten Dampfes als im HD-Teilabschnitt/Kreislauf ist hinreichend, um die Heizaufgaben im ND-Teilabschnitts/Kreislauf auf niedrigerem Temperaturniveau zu erfüllen. In analoger Weise liegt der Druck im MD-Teilabschnitt/Kreislauf zwischen dem ND- und dem HD-Teilabschnitt/Kreislauf.

Unter dem HD-Teilabschnitt/Kreislauf ist vorliegend der Teilabschnitt/Kreislauf des Verteilerkreislaufes zu verstehen, in dem (innerhalb des Verteilerkreislaufs) der maximal mögliche Enddruck und damit in der Regel auch höchste Endtemperatur des Verteilerkreislaufs im Medium erzeugbar ist, was aufgrund der Leistung und/oder Anzahl an (insb. seriell zueinander geschalteten) Verdichtern und/oder der hiermit möglichen Verdichterleistung erfolgen kann.

Jeder Teilabschnitt weist eine eigene Verteilleitung 572.1 auf und ist über eine hinführende Leitung oder Leitungsast 576, 577 und 578 als Abzweigung vom hinführenden Leitungsast 571 mit dem Verdampfer 172 verbunden. Weiterhin führt eine gemeinsame Sammelleitung 572.2, in den jeder Teilabschnitt einspeist, in den rückführenden Leitungsast 573. Die Wärmetauscher innerhalb eines jeden dieser Teilabschnitte sind zueinander parallelgeschaltet, sofern zwei oder mehr Wärmetauscher umfasst sind.

Hierbei ist der Leitungsknoten, mit dem die erste (Ab-)Leitung 576 von dem zentralen Leitungsast 571 abzweigt zwischen dem ersten Verdichter 192 und dem zweiten Verdichter 193 angeordnet, der Leitungsknoten, mit dem die zweite (Ab-)Leitung 577 vom zentralen Leitungsast 571 abzweigt, ist zwischen dem zweiten Verdichter 193 und dem dritten Verdichter 194 angeordnet und die dritte (Ab-)Leitung 578, die als letzte Teilstück des zentralen Leitungsastes 571 darstellt, schließt sich stromabwärts zum dritten Verdichter 194 an. Hierbei versorgt die ersten (Ab-)Leitung 576 alleinig den Wärmetauscher 302, den ND-Teilabschnitt des Leitungsabschnitts 572, die zweite (Ab-)Leitung 577 die beiden parallel geschalteten Wärmetauscher 322, 328, den MD-Teilabschnitt des Leitungsabschnitts 572, und die dritte (Ab-)Leitung 578 die drei parallel geschalteten Wärmetauscher 342, 352, 365, den HD-Teilabschnitt des Leitungsabschnitts 572. Somit weisen die drei (Ab-)Leitungen 576, 577 und 578 jeweils unterschiedliche Druckniveaus und unterschiedliche Temperaturniveaus auf. Die Einspeisung von flüssigem Medium über die (Sumpf-)Leitung 574 bzw. über die Abzweigungen in den zentralen Leitungsast 571, erfolgt analog zur Ausführung der Figur 2, wobei die Leitungsknoten der drei (Ab-)Leitungen in Strömungsrichtung vor dem Leitungsknoten der jeweiligen Einleitung aus der (Sumpf-)Leitung 574 angeordnet sind.

Der in der Darstellung untere Teilabschnitt, in den die Wärmetauscher 342, 352, 362 eingebunden sind, ist über die Druckregeleinheit 585 in der Sammelleitung 572.2, und der mittlere Teilabschnitt, in den die Wärmetauscher 322, 328 eingebunden sind, ist über die Druckregeleinheit 586 in der Sammelleitung 572.2 auf einem eigenständigen Druckniveau gehalten. In der Sammelleitung 572.2 erfolgt somit eine gestufte Entspannung.

Die drei Teilabschnitte des Leitungsabschnitts 572, über den die Energieverteilung an als Wärmesenken arbeitende Wärmetauscher erfolgt, münden in eine gemeinsame (Rück-)Leitung 573 und in den Verdampfer 172.

Vorteil dieser Ausführungsvariante gegenüber der aus Figur 2, ist der deutlich geringere Energiebedarf für den zweiten und dritten Verdichter 193, 194.

Der wasserführende Verteilerkreislauf weist am hinführenden Leitungsast 571 unmittelbar stromabwärts zum zweiten Verdampfer 172 ebenfalls eine Temperatur von 130 °C und 2,7 bar auf. Nach dem ersten Verdichter 192 beträgt der Druck 5,4 bar, der mit einem Energieverbrauch von ebenfalls 238 kW erreicht wird, nach dem zweiten Verdichter 194 beträgt der Druck ebenfalls 10,8 bar, der aber mit einem elektrischen Energieverbrauch von nur noch 145 kW erreicht wird und nach dem dritten Verdichter 194 beträgt analogerweise der Druck 20 bar bei einer Temperatur von 250 °C, allerdings erreicht durch elektrischen Energieverbrauch von nur noch 115 kW für den dritten Verdichter 194. Dieser Vorteil stellt sich ein, weil der im zweiten und dritten Verdichter 192, 193 stark verringert ist. Neben dem energetischen Vorteil ist hiermit verbunden, dass der zweite und dritte Verdichter 193, 194 baulich wesentlich kleiner ausgelegt werden können, was weiterhin in der Regel die Wartung und den Betrieb bzw. die Aufstellung ebenfalls verbessert.

Zur Versorgung der in der Figur 3 gezeigten Wärmetauscher, die insb. die Sumpfwärmetauscher der Trennkolonnen sind, muss ein sehr hohes Temperaturniveau bereitgestellt werden, so dass über den Bedarfs- und Regelungswärmetauscher 199 das zirkulierende Medium wieder gekühlt werden muss. Die erforderliche Kühlleistung beträgt im gezeigten Beispiel ca. 368 kW, die in der (Rück-)Leitung 573 vor der Druckregelungseinheit 195 bzw. vor Eintritt in den zweiten Verdampfer 172 bereitgestellt werden muss.

Grundsätzlich könnten in den ND-Teilabschnitt, in den der Wärmetauscher 302 eingebunden ist, auch die Wärmetauscher 206 der Reaktionseinheit 102 und/oder der Wärmetauscher 158 der Konditionierungseinheit 104 eingebunden werden.

Im gezeigten Beispiel der Figur 3 sind die Wärmetauscher 304 (Kondensator) zur EK als Wärmequelle mit den beiden als Wärmesenke arbeitenden Wärmetauscher 206, 158 gekoppelt. Hierbei ist der Wärmetauscher 206 im Vorlauf des Nachreaktors 210 (nicht dargestellt) und Wärmetauscher 158 in die (Feed-)Leitung 153 stromaufwärts zum Hauptreaktor 200 eingebunden. Diese Option der EK einer direkten Wärmekopplung ist unten links in der Figur 3 dargestellt. Zum besseren Verständnis sind der Wärmetausche 206 und die Leitung 116 somit zweifach in dieser Figur 3 dargestellt. Diese selektive Direktverschaltung und Verteilung der Wärme des Kondensators 304, hat gegenüber der Einbindung der Wärmesenken in den Verteilerkreislauf 570 gem. bspw. Fig. 2 oder Fig. 3, wie vorstehend beschrieben, eine Effizienzsteigerung von 30 bis 40 % zur Folge, insb. wenn mind. eine EK eine integrierte stoffbasierte EK ist, indem Wärmetauscher 304 mit dem (Vorlauf-)Wärmetauscher 158 und/oder dem (Feed-)Wärmetauscher 327 (nicht dargestellt; analog Fig. 6) stromaufwärts zur ersten Trennkolonne 320 der ersten Trennstufe 106B gekoppelt wird.

Vorliegend meint "Effizienzsteigerung", wenn nichts Abweichendes ausgeführt wird, einen geringeren Energieverbrauch. Der Bezug ergibt sich aus dem Zusammenhang und kann bezogen sein auf die Anlage, den jeweils beschriebenen Anlagenabschnitt oder den verbesserten Apparat bspw. durch Integration von zwei Wärmetauschern in einen einzigen.

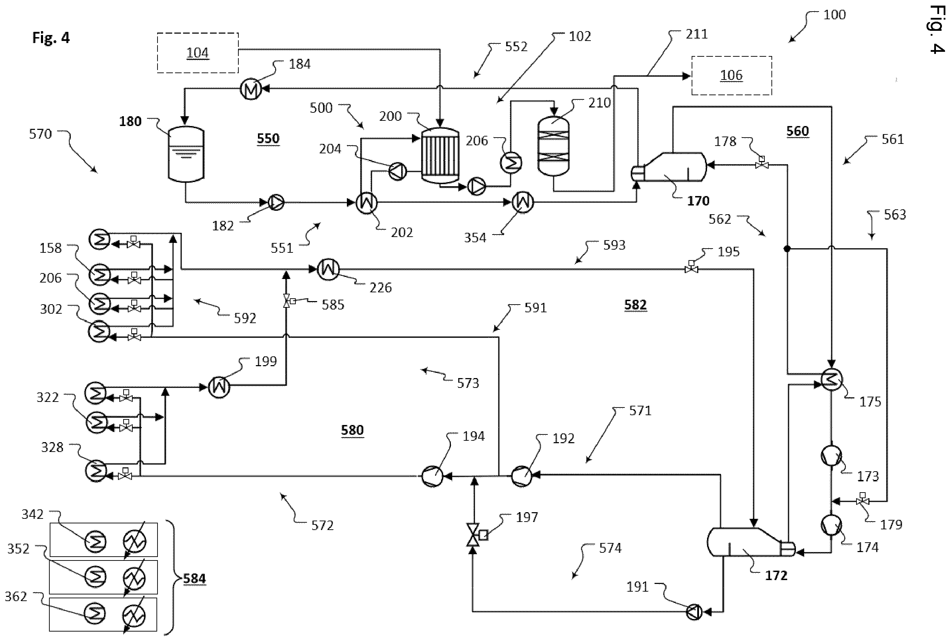

Das Ausführungsbeispiel, wie in der Figur 4 gezeigt ist, kann als Alternative oder Verbesserung zum Ausführungsbeispiel der Figur 3 gesehen werden, weil die HD-Teileinheit alternativ gelöst wurde. In dem Ausführungsbeispiel der Figur 4 umfasst der Verteilerkreislauf 570 zwei Teilkreisläufe. Diese Teilkreisläufe sind ein Mitteldruck-Verteilerkreislauf 580 (MD-Verteilerkreislauf) und ein Niederdruck-Verteilerkreislauf 582 (ND-Verteilerkreislauf). Der MD-Verteilerkreislauf 580 ist im Wesentlichen analog zur Ausführungsform des MD-Teilabschnitts der Figur 3 ausgebildet. Dieser ist stromabwärts zum zweiten Verdichter 194 angeordnet und es ist ebenfalls auch nur eine Zwischeneinspeisung von einem Medienstrom über die (Sumpf-)Leitung 574 zwischen die beiden Verdichter 192, 194 vorgesehen. In der abzweigenden Leitung des ND-Verteilerkreislaufs 582 beträgt der Druck nach dem zweiten Verdichter 194 ca. 8 bis 12 bar, vorliegend ca. 10,8 bar. Der ND-Verteilerkreislauf 582 ist im Wesentlichen analog zur Ausführungsform des ND-Teilabschnitts der Figur 3 ausgebildet. Dieser ist stromabwärts zum ersten Verdichter 192 angeordnet. In der abzweigenden Leitung 591 des ND-Verteilerkreislaufs 582 beträgt der Druck ca. 5 bis 6 bar, vorliegend 5,4 bar.

Der WT-Teil des Verdampfers 172 ist analog eingebunden in den Zwischenkreislauf 560, in dem ein leichtflüchtiges Medium, wie bspw. Methanol oder Butanol im Kreis strömt.

Der ND-Verteilerkreislauf 582 umfasst einen erste, hinführenden Leitungsast 591, einen zweiten verteilenden Leitungsast 592 (auch Leitungsabschnitt 592 genannt), der in einen zentralen, rückführenden Leitungsast 593 (auch (Rück-)Leitung 593 genannt) mündet. Wie ausgeführt, zweigt der hinführende Leitungsast 591, in dem die Wärmetauscher 158, 206, 302 und ggf. weitere Wärmetauscher als Energiesenke in einer Parallelverschaltung eingebunden sind, stromabwärts zum ersten Verdichter (192) aus dem Leitungsast 571ab. Die (Rück-)Leitung 593 bildet die gemeinsame Rückleitung für den MD- und den ND-Verteilkreislauf 580, 582 hin zum K-Teil des zweiten Verdampfers 172. In die (Rück-)Leitung 593 ist ein Wärmetauscher 226 eingebunden, über den eine erforderliche Rücklauftemperatur für den Betrieb des Verdampfers 172 und/oder Aufwärmschritte vor oder beim Start des Betriebs der Anlage 100 bzw. des Verfahrens vorgenommen werden können. Weiterhin ist eine insb. steuerbare Druckregeleinheit 195 in die (Rück-)Leitung 593 eingebunden, um das niedrigere Druckniveau im K-Teil des Verdampfers 172 von ca. 2,5 bis 3,5 bar bei ca. 125 bis 130 °C sicherzustellen.

Um den Wirkungsgrad der Anlage und der zusammenwirkenden drei bzw. vier Kreisläufe insgesamt auf einem vorteilhaften Niveau zu halten, ist eine Wärmetauschergruppe 584 (WT-Gruppe 584) nicht eingebunden und vorteilhafterweise elektrifiziert. Die Wärmetauscher 342, 352, 362 der drei zugehörigen Sumpfkreisläufe von Trennkolonnen sind somit mit einer elektrischen Heizung beheizt, ohne Leitungsverbindung für ein fließendes Wärmetauschmedium. Die Motivation hierfür ist, dass der Bedarf an elektrischer Leitung für den Betrieb des dritten Verdichters in einer vergleichbaren Größe wie die direkte Beheizung wäre, so dass es zur Vermeidung von Wartungsaufwand, Investitionskosten etc. sinnvoll ist, bspw. einen geringfügig höheren Energieverbrauch für die elektrische Direktbeheizung für die elektrische WT-Gruppe 584 vorzusehen und die vorstehend genannten Nachteile zu vermeiden. Weiterhin vereinfacht sich auch der konstruktive, bauliche Aufwand bei den betroffenen Trennkolonnen, weil nur eine elektrische Spannungsversorgung sichergestellt werden muss und auch das Bauvolumen eines elektrisch beheizten Wärmetauscher wesentlich unterhalb von dem eines medienführenden Wärmetauschern liegt.

Der Vorteil der Aufteilung des Verteilerkreislaufs 570 in Teilkreisläufe 580, 582, ist dass diese Teilkreisläufe auf unterschiedlichen Druck- und Temperaturniveaus betrieben werden können und hierüber der Verbrauch an elektrischer Energie für die Verdichter, insb. die Verdichter nach dem ersten Verdichter, verringert, sowie deren Baugröße parallel verkleinert werden können. Durch die elektrische Direktbeheizung kann schließlich ein Verdichter vollständig entfallen, insb. der mit dem geringsten Wirkungsgrad, mit Blick auf den Temperaturanstieg im Medium pro kW elektrischer Leistung für den Verdichter. Bei dieser Ausführungsform wird durch den ND- und MD-Verteilerkreislauf 580, 582 eine Minimierung der Stromkosten für den Betrieb der Verdichter erreicht, da die Temperaturniveaus der Kreisläufe an die benötigte Temperatur der Wärmesenken angepasst sind und somit unnötige Verdichtungsstufen vermieden und/oder einer jeweilige Verringerung des zu verdichtenden Dampfvolumenstroms erreicht wird.

Das Ausführungsbeispiel, wie es in der Figur 5 gezeigt ist, zeigt eine zur Figur 2 analoge Ausführungsform, wobei der Verteilerkreislauf 570 stark vereinfacht ausgeführt ist, in dem Verteilerkreislauf 570 ist nur ein Wärmetauscher, hier der Wärmetauscher 302 aus dem Sumpfumlauf des Trennkessels 300 mit einem für die Anlage 100 sehr hohen oder höchsten Energiebedarf eingebunden ist. Der Verteilerkreislauf 570 konnte weiterhin, aufgrund der Einbindung des nur einen Wärmetauschers 302 bezüglich der Verdichter im ersten Leitungsast 571 vereinfacht werden, indem nur eine Verdichter 192 eingebunden wurde.

Grundsätzlich könnten in den Verteilerkreislauf 570, in den der Wärmetauscher 302 eingebunden ist, auch die Wärmetauscher 206 der Reaktionseinheit 102 und/oder der Wärmetauscher 158 der Konditionierungseinheit 104 eingebunden werden.

Im gezeigten Beispiel der Figur 5 ist die Option gezeigt, bei der der Wärmetauscher 304 (Kondensator) als Wärmequelle mit dem Wärmetauscher 206, dem Vorlauf des Nachreaktors 210 (nicht dargestellt) und Wärmetauscher 158, eingebunden in die (Feed-)Leitung 153 stromaufwärts zum Hauptreaktor 200, als Wärmesenken direkt verbunden ist. Diese Option einer Direktverschaltung von einzelnen Wärmetauschern ist unten links in der Figur 5 dargestellt. Zum besseren Verständnis sind der Wärmetausche 206 und die Leitung 116 somit zweifach in dieser Figur 5 dargestellt. Diese selektive Direktverschaltung und Verteilung der Wärme des Kondensators 304, hat gegenüber der Einbindung der Wärmesenken in den Verteilerkreislauf 570 gem. Fig. 2 oder Fig. 3, wie vorstehend beschrieben, eine Effizienzsteigerung von 30 bis 40 % zur Folge, insb. wenn mind. eine EK eine integrierte stoffbasierte EK ist, indem Wärmetauscher 304 mit dem (Vorlauf-)Wärmetauscher 158 und/oder dem (Feed-)Wärmetauscher 327 (nicht dargestellt; analog Fig. 6) stromaufwärts zur ersten Trennkolonne 320 der ersten Trennstufe 106B gekoppelt wird.

Die Ausführungsform, in der nur der Wärmetauscher 302 im dem Verteilerkreislauf 570 vorgesehen wird, stellt ein besonders einfaches und effizientes Übertragungssystem dar, da nur eine Verdichterstufe bzw. Verdichter benötigt wird, um einen erheblichen Teil des Wärmebedarfs mit hoher Effizienz zu decken. Allein durch diese Integration kann eine Effizienzsteigerung gegenüber einer Direktbeheizung der Wärmetauscher von ca. 40% erreicht werden. Die direkte Verschaltung der Wärmetauscher 304 als Wärmequelle mit den Wärmetauschern 206, 158 als (Verbraucher) Wärmesenken, führt zu einer Effizienzsteigerung von ca. 28%, d.h. Energieeinsparung von 28 %. Somit können durch beide Maßnahmen, den Verteilerkreislauf 570 und die Direktverschaltung des Kondensators 304 mit den Wärmetauschern 206, 158 eine Effizienzsteigerung von ca. 70 % erreicht werden.

Figur 6 zeigt eine weiter optionale Ausführungsform . Hierbei ist zur Entlastung und Steigerung des Wirkungsgrades der verschalteten geschlossenen Medienkreisläufe 550, 560, 570, stromaufwärts zur ersten Trennkolonne 320 ein (Vorlauf-)Wärmetauscher 327 vorgesehen. Vorteilhafterweise wird der abfließende Medienstrom des (Vorlauf-)Wärmetauscher 206 des Nachreaktors 210, das ein Temperaturniveau von ca. 110 bis 125 °C aufweist, als Heizmedium diesem (Vorlauf-)Wärmetauscher 327 zuführt. Auf diese Weise wird der Energiebedarf des Wärmetauschers 322 im Sumpfumlauf der ersten Trennkolonne 320 und damit der des Verteilerkreislaufs 570 gesenkt. Ein besonderer Vorteil stellt sich ein, wenn eine integrierte stoffbasierte EK des Wärmetauscher 304 mit dem (Feed-)Wärmetauscher 327 stromabwärts der Druckregeleinheit 222 und stromaufwärts zur ersten Trennkolonne 320 der ersten Trennstufe 106B (nicht dargestellt) vorgenommen wird.

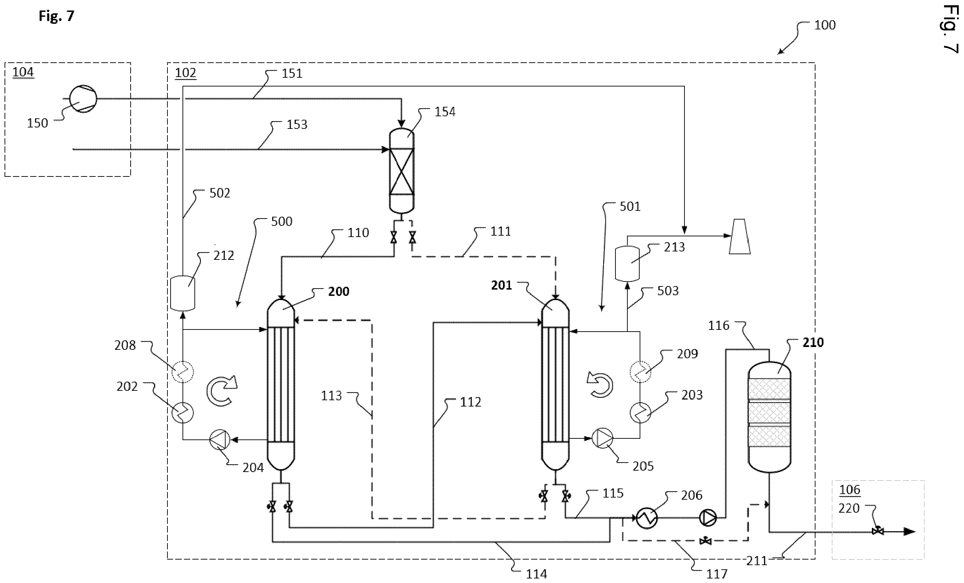

Figur 7 zeigt eine verbesserte Variante der Reaktoreinheit 102. Hierbei sind zwei Hauptreaktoren 200, 201 schaltbar zueinander in Reihe geschaltet. Die steuer- und/oder regelbaren Ventile/- einheiten sind zum Teil in der Figur 5 eingezeichnet, weitere können vom Fachmann bedarfsweise vorgehen werden, um den sicheren Betrieb der beiden Hauptreaktoren 200, 201 sicherzustellen. Die beiden Hauptreaktoren 200, 201 sind jeweils in einen Kühlkreislauf 500, 501 eingebunden, mit dem jeweils der Festbettreaktor 200, 201 auf einer zulässigen, gekühlten Reaktionstemperatur gehalten wird. In der darstellten und mit vollausgezogenen Linien dargestellten Linien dargestellten Schaltung der Hauptreaktoren 200, 201 wird der erste Hauptreaktor 200 über die Leitung 110 kommend vom Mischbehälter 154 zuerst angeströmt und der erste Reaktionsanteil erfolgt in diesem Hauptreaktor 200. Stromabwärts über einen unteren Auslass und die Leitung 112 wird das Stoffgemisch in den Kopf des zweiten Hauptreaktors 201 geleitet und verlässt diesen über einen Bodenauslass und Leitung 115 in die gemeinsame Leitung 116 als Feed in den gemeinsamen Nachreaktor 210. In die gemeinsame Leitung 116 ist eine Wärmetauscher 206 eingebunden, worüber das für den Nachreaktor 210 erforderliche Temperaturniveau sichergestellt werden kann. Die Reihenfolge der Durchströmung der beiden Hauptreaktoren kann auch in umgekehrter Reihenfolge vom Hauptreaktor 201 zum Hauptreaktor 200 erfolgen. Hierzu wird in analoger Weise der Hauptreaktor 201 über die Leitung 111 zuerst angeströmt, wobei die Leitung 110 zum Kopf des Hauptreaktors 200 verschlossen ist. Das Stoffgemisch verlässt diesen Festbettreaktor über einen Bodenauslass und die Leitung (Zwischen-)Leitung 113, die mit dem Kopf des Hauptreaktors 200 verbunden ist, wobei die Leitung 115 verschlossen ist. Schließlich verlässt das Stoffgemisch den Hauptreaktor 200 über einen Bodenauslass und Leitung 114, die analog in die gemeinsame Leitung 116 mündet und damit zum Nachreaktor 210 führt. Aus beiden Kühlkreisläufen 500, 501 zweigt jeweils eine Leitung 502, 503 ab, die über jeweils einen Behälter 212, 213 geführt ist, die als Druckausgleichsbehälter dienen, und weiter zu einem Kamin und/oder einer vollständigen Oxidationseinheit. Die Leitungen und Ventile/-einheiten sind derart vorhanden und ausgelegt, dass jeder der Hauptreaktoren 200, 201 alleinige betrieben werden kann und der Stoffstrom um den jeweils anderen Hauptreaktor vollständig vorbeigeleitet werden kann. Die Fließrichtung der beiden Kühlkreisläufe 500, 501 ist durch einen Pfeil symbolisiert, wobei die beide Kühlkreisläufe 500, 501 vorteilhafterweise identisch oder im Wesentlichen gleich ausgebildet sind, da die beiden Hauptreaktoren 200, 201 abwechselnd bzgl. der Strömungsrichtung des Edukt- bzw. des Stoffstroms als erster bzw. zweiter Hauptreaktor betrieben werden.

Die Kühlkreisläufe 500, 501 sind allerdings derart ausgelegt und regelbar, dass je nach verfahrenstechnischen Bedarfen, wie insb. Produktleitung und/oder Produktqualität, jeweils eigenständige Kühlleistungen oder Kühlaufgaben erfüllt werden. Dies betrifft insb. den Volumenstrom pro Zeiteinheit an Kühlmedium und/oder das Temperaturniveau bzw. die zulässige Erwärmung des jeweiligen Kühlmediums.

In der Figur 7 ist weiterhin eine optionale Leitung 117 als gestrichelte Linie (Bypass 2) dargestellt, womit der Nachreaktor 210 umgangen werden kann, wenn beispielsweise dieser gewartet werden muss und/oder die Katalysatorfüllung erneuert werden muss. In diesem Fall wird mind. der in Strömungsrichtung des Stoffstroms zweite Hauptreaktor derart temperiert, dass die vollständige Reaktion bei der gewünschten Produktqualität, insb. dem gewünschten Anteil an den jeweiligen Isomeren sichergestellt ist. Der Leitungsabzweig der Leitung 117 kann in Strömungsrichtung vor oder nach Wärmetauscher 206 vorgesehen werden, wobei es vorteilhaft ist, diesen stromaufwärts zu Wärmetauscher 206 vorzusehen, um diese ggf. auch bypassen zu können und nötige Wartungsarbeiten bei laufender Anlage vornehmen zu können.

Die Umgehung des Hauptreaktors 200 kann im Betrieb des rechts im Bild dargestellten Hauptreaktors 201 über die Leitungen 111, 115 und 116 erfolgen. Die Umgehung des Hauptreaktors 201 kann im Betrieb des links im Bild dargestellten Hauptreaktors 200 über die Leitungen 110, 114 und 116 erfolgen, so dass jeweils die (Kreuz-)Leitungen 112, 113 zwischen den beiden Hauptreaktoren 200, 201 nicht durchflossen werden.

In den Kühlumläufen 500, 501 sind weiterhin (optional) bei der dargestellten Anlagenvariante Wärmetauscher 208, 209 eingebunden. Dieser werden mit einem Heizmedium betrieben, insb. Dampf, und dienen im Anfahrschritt der Hauptreaktoren 200 der (Vor-)Temperierung auf Reaktionstemperatur der jeweiligen Hauptreaktoren 200, 201. Das Niveau dieser Vortemperierung liegt bei ca. 80 bis 100 °C, idealerweise 85 °C bis 95 °C. In dem gezeigten Anlagen- und Verfahrensbeispiel, wie bereits zur Figur 1 ausgeführt, ist es bei dem Ziel, ein möglichst niedriges trans/trans-Isomerenverhältnis von ca. 17 bis 23 Gew.% zur realisieren vorteilhaft, wenn die mit Katalysator neu befüllten Hauptreaktor 200, 201 auf eine Temperatur von ca. 90 °C mittels des jeweiligen Wärmetauschers 208, 209 vorgeheizt werden, bzw. der jeweilige neu befüllte Hauptreaktor 200, 201 entsprechend vorbeheizt wird.

Durch die Vorheizung auf ca. 85 bis 95 °C wird ermöglicht, dass die Reaktion bei dem vorliegenden Katalysator unmittelbar starten kann, ohne oder im Wesentlich ohne Recyclingströme des Stoffgemisches der Prozesses bis zum Erreichen der gewünschten Reaktionstemperatur.

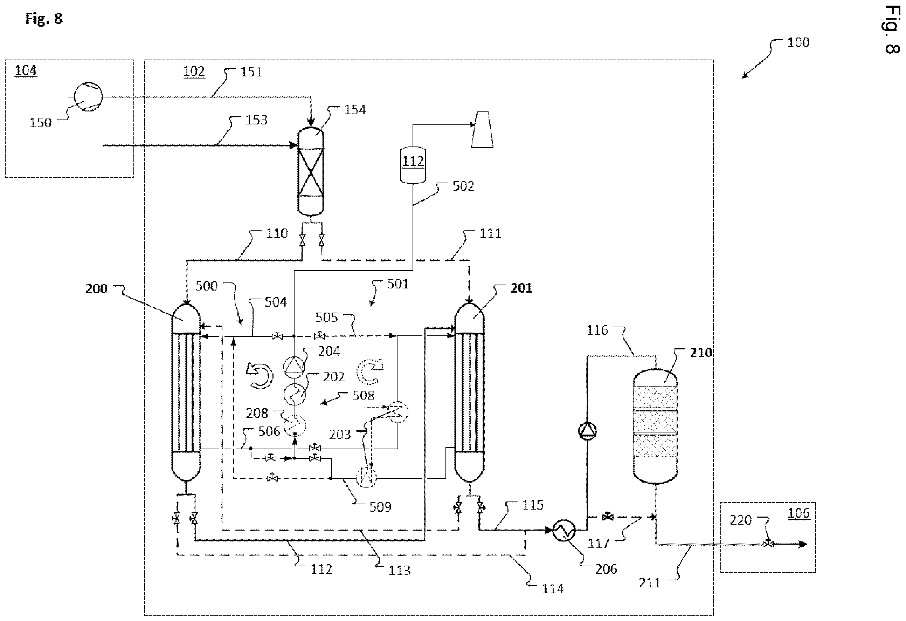

Die Variante der schaltbaren Hauptreaktoren 200, 201, wie sie in der Figur 8 dargestellt ist, unterscheidet sich zu der gem. Figur 7 darin, dass der Kühlkreislauf 500 des ersten Hauptreaktors 200 mit dem Kühlkreislauf des zweiten Hauptreaktors 201 ebenfalls in Reihe geschaltet ist. Hierbei ist nur ein (kühlender) Wärmetauscher 202 und nur eine Pumpe 204 für die gemeinsame Kühlung und den Medienumlauf vorgesehen, so dass der gemeinsame (zentrale) Leitungsast 508 in beiden Kühlkreisläufen 500, 501 in der Regel ständig und nur in eine Richtung durchströmt wird, unabhängig von der Verschaltung der beiden Hauptreaktoren 200, 201. Hierbei muss der "zentrale" Leitungsast natürlich nicht faktisch zwischen den beiden Hauptreaktoren 200, 201 angeordnet sein. Gezeigt ist die Variante als durchgezogenen Linien, bei der der erste Hauptreaktor 200 (links) zuerst mit dem Eduktgemisch über die Leitung 110 beschickt wird und auch die (Kühlmedien-)Einleitung nach dem Wärmetauscher 202 und der Pumpe 204 zuerst über diesen Hauptreaktor 200 erfolgt. Die in diese Schaltung nicht medienführenden Leitungen oder das Stoffgemisch führenden Leitungen sind gestrichelt dargestellt. Auch bei dieser Varianten besteht die Möglichkeit, das Stoffgemisch und/oder das (Kühl-)Medium vollständig an dem jeweils anderen Hauptreaktor vorbeizuleiten. Somit kann bspw. im Befüllvorgang eines der beiden Hauptreaktoren 200, 201, der jeweils andere unter maximaler Leistung weiter betrieben werden. Der Bypass des Stoffstroms bzw. des jeweiligen Hauptreaktors 200, 201 ergibt sich analog zu Figur 7.

In der gezeigten Beispielschaltung wird der zentrale Leitungsast 508 und der Wärmetauscher 202 durchflossen und über die Leitung 504 im Gleichstrom in den Medienraum ersten Hauptreaktors 200 geleitet, wobei zum gemeinsamen Leitungsknoten führende Leitung 505, kommend vom zweiten Hauptreaktor 201, gesperrt ist. Über die Leitung 506 verlässt das Medium an einem tiefen Auslass den ersten Hauptreaktor 202 und wird zu einem hohen Einlass in den Medienraum des zweiten Reaktors 201 geleitet (Kreuzleitung). Das Medium durchfließt den Medienraum des zweiten Hauptreaktors 201 ebenfalls im Gleichstrom und verlässt diesen an einem tiefliegenden Auslass über die Leitung 509, von der eine Abzweigung wieder in den zentralen Leitungsast 508 mündet, so dass der Kreislauf erneut durchflossen werden kann. Analog wird über die Leitung 505 der rechts dargestellte Hauptreaktor 201 zuerst durchströmt, wenn die Leitung 504 gesperrt ist. Das Medium verlässt dann den Medienraum des zweiten Hauptreaktors 201 über die Leitung 509 und wird an einem hochgelegenen Einlass in den Medienraum des anderen Hauptreaktors 200 geleitet. Der Auslass des Medienraums an einem tiefgelegenen Punkt führt in die Leitung 506 und von dort über eine Abzweigung in den zentralen Leitungsast 508

Der besondere Vorteil des schaltbaren Hauptreaktors 200, 201 besteht in der Möglichkeit, den zuerst angeströmten Hauptreaktor bei einer höheren Temperatur zu betreiben, weil der Katalysator schon teilweise erschöpft und inaktiviert ist, im Wesentlichen ohne hierbei das trans/trans-Isomerenverhältnis negativ zu beeinflussen (d.h. zu erhöhen). Parallel hierzu fällt stärker erwärmtes Kühlmedium im jeweiligen (Umlauf-)Wärmetauscher 202, 203 des zuerst durchflossenen Hauptreaktors 200, 201 an. Dieses heißere Wärmetauschmedium kann aufgrund der höheren Temperatur besser in der Anlage zur integrierten medienbasierten EK und/oder zum üblichen Wärmetausch mit einem Stoff- Eduktstrom eingesetzt werden.

In der Figur 8 ist analoge zur Figur 7 die optionale Leitung 117 als gestrichelte Linie (Bypass 2) dargestellt, womit der Nachreaktor 210 umgangen werden kann, wobei die Leitung 117 stromabwärts zum (Vorlauf-)Wärmetauscher 206 des Nachreaktors 210 abzweigt.

In der Figur 8 ist weiterhin eine Anlagenvariante (gestrichelt) dargestellt, indem zur Erlangung einer höheren Sicherheit bzw. eines höheren Freiheitsgrades des Temperaturmanagements in der jeweiligen (Kreuz-)Leitung 504, 505 der Kühlkreisläufe 500, 501 bedarfsweise schaltbare und regelbare Wärmetauscher 203 (Vorlaufkühler) angeordnet sind. In dem gezeigten Ausführungsbeispiel sind die beiden Wärmetauscher 202 in Reihe geschaltet, da durch die jeweils inaktive (Kreuz-Leitung, indem gezeigten Beispiel die Leitung 505, am Einbauort für den Wärmetauscher 202 keine Erwärmung erfolgt. Die Kühlmedienleitung bzw. der Kühlmedienkreislauf der Wärmetauscher 202 kann bei einer vorteilhaften Variante in einem Seitenstrom bzw. über einen Neben- oder Hilfskreislauf über die Pumpe 204 betrieben werden.

Der (Vorlauf-)Wärmetauscher 206 ist vorliegend vorrangig beschrieben und teilweise in einer EK gezeigt, bei denen dieser als Wärmetauscher 206 vorrangig als Wärmesenke arbeitet, also der darin geführte Stoffstrom erwärmt wird. Durch die an den Hauptreaktor 200, 201 angepasste, abhängige Betriebsweise des Nachreaktors 210 kann mind. zeitweise, insb. dauerhaft, eine Kühlung des Stoffstroms in der (Feed-)Leitung 116 stromaufwärts zum Nachreaktor 210 erforderlich sein, weil im (adiabatischen) Nachreaktor ca. 10 bis 20% des Umsatzes erfolgen, so dass eine Erwärmung des Stoffstroms gemessen im Auslass bis auf ca. 140 °C erfolgt. Somit kann unabhängig von den hierhin beschriebenen Ausführungsformen und Varianten der Anlagen und des Verfahrens eine angepasste EK zur Kühlung (Wärmetauscher 206 arbeitet als Wärmequelle) vorgesehen werden. Alternativ oder zusätzlich kann eine ergänzende Kühlung über eine geänderte oder weitere EK vorgesehen werden.

Der Vorteil dieses Seitenstroms oder Neben-/Hilfskreislaufes eines Kühlmittels über die Wärmetauscher 203 hat den großen Vorteil, dass diese zusätzliche Kühlleistung nur bedarfsweise abgerufen werden braucht und mit geringem Aufwand eine weitergehend, bedarfsmäßige Regelung der Temperatur im jeweils zweiten beiden seriell geschalteten Hauptreaktoren erfolgen kann.

Insgesamt sind eine Vielzahl von üblichen, dem Fachmann bekannte und für eine vorteilhafte Prozessführung nötige oder sinnvolle Steuer- und Regelungselemente nicht dargestellt, wie Sensoren (Durchfluss, Temperatur, Druck etc.), Anzeigen, Stell- und Regelglieder (insb. Ventile, weitere Pumpen, Verdichter), Sammelbehälter etc. und sind bedarfsweise zu ergänzen. Insb. sind auch bei der Nennung von "einer" Pumpe" oder "einem" Verdichter übliche Redundanzen aus mind. zwei parallelen Aggregaten gemeint, insb. von zwei parallelen Pumpen oder zwei parallelen Verdichtern. In analoger Weise ist "ein Wärmetauscher" nicht limitierend zu verstehen und meint mit der jeweiligen, örtlichen Wärmetauschaufgabe auch Anordnungen von in Reihe geschalteten Wärmetauschern oder parallel geschaltete, auch redundanten, Wärmetauschern, wobei hiermit keine Verschaltungen mit mind. einem weiteren Wärmetauscher und örtlich unterschiedlicher Wärmetauschaufgabe zu verstehen ist.

Grundsätzlich sind alle Wärmetauscher und Kondensatoren derart ausgebildet, dass ein indirekter Wärmeübertrag erfolgt und keine stoffliche Vermischung von Edukten, Produkt, Nebenprodukten und/oder Lösungsmittel mit den (Heiz-/Kühl-)Medium, wie Gas, Dampf, Wasser, Öl, Sole (Brine) etc. erfolgt, es sei denn es ist etwas hiervon Abweichendes ausgeführt.

Auch wenn vorliegend Bauteile, wie Ventile, Druckregeleinheit, Absperraggregate etc. zur vereinfachten Beschreibung einzeln oder gesondert dargestellt sind und zum Teil nicht einzeln genannt wurde, ist dies nicht limitierend zu lesen, vielmehr kann der Fachmann bedarfsweise zwei oder mehrere dieser Bauteile in einer Ventil- oder Stelleinheit zusammenfassen oder Mehrwegventile stattdessen vorsehen.

Vorliegend meint "stromaufwärts" oder "stromabwärts" die Anordnung und/oder Fließrichtung des produktreichen Stoffstroms, wenn nichts hiervon Abweichendes ausgeführt ist. Weiterhin ist unter "Medien", "Medienstrom", "Medienleitung" etc. immer ein Heiz- oder Kühlmedium bzw. die zugehörige Leitung gemeint, wenn nichts hiervon Abweichendes ausgeführt ist.

Die Begriffe "Sumpfablauf", "Sumpfauslauf", "Sumpfableitung" oder "Sumpfausleitung" werden zum Teil synonym verwendet, ebenso werden in analoger Weise die Begriffe "Kopfablauf, - auslauf, - ableitung oder -ausleitung" zum Teil synonym verwendet.

Weiterhin ist der Begriff "Wärmetauscher, der als Kondensator betrieben wird" breit auszulegen, und meint auch eine unvollständige Kondensation oder Abkühlung des zugeleiteten Stoffstroms, so dass ein "Wärmetauscher, der als Kondensator betrieben wird", auch teilweise synonym als "Kondensator" benannt wird.

Der Begriff "Sumpfpumpe" meint eine in einen Sumpfumlauf eines Apparates (Trennkolonne, Behälter, etc.) eingebundene Pumpe und/oder eine sich stromabwärts zum Sumpfauslass eines Apparates befindliche Pumpe, die zur Förderung flüssigen Stoffstroms vorgesehen ist.

Für alle Ausführungsformen und Varianten der Anlage und des Verfahrens kann generell vorgesehen werden, dass die zweite Trennkolonne 340 und die dritte Trennkolonne 350 als eine einzige Kolonne ausgeführt werden, insb. als eine Trennwandkolonne (nicht dargestellt). Hierbei können vorteilhafterweise die Trennaufgaben der zweite Trennkolonne 340 und der dritten Trennkolonne 350 mind. teilweise, idealerweise vollständig mittels der Trennwandkolonne (nicht dargestellt) ausgeführt werden, wie sie bspw. aus den Druckschriften EP 012 62 88 B1 oder EP 012 23 67 A2 bekannt sind.

Insgesamt kann ein großer, energetischer Vorteil mit der erfindungsgemäßen Anlage und dem Verfahren erreicht werden, der darin besteht, dass eine starke Verringerung der extern zugeführten Energieströme ermöglicht wurde, insb. die Einsparung von großen Mengen an (externem) Heizdampf.

## Patentansprüche

1. Anlage (100) zur Hydrierung von Methylendianilin (MDA; Edukt1) mit einem Wasserstoffgeber (Edukt2), insb. einem gasförmigen Wasserstoffgeber, vorzugsweises Wasserstoff (H2)
umfassend eine Konditionierungseinheit (104) für die Edukte, eine Reaktoreinheit (102) zur Synthese von PACM und eine Trenneinheit (106), wobei
- die Konditionierungseinheit (104) (Zu-)Leitungen für Edukte1, Edukt2 und mind. ein Lösungsmittel, mind. einen Wärmetauscher (158) in mind. einer (Zu-)Leitung, mind.
einen Mischer (152) zur Mischung der Edukte und/oder mind. eines Edukts mit mind. einem Lösungsmittel umfasst;
- die Reaktoreinheit (102) mind. einen Festbettreaktor als Hauptreaktor (200, 201) mit einer immobilen Katalysatorpackung umfasst, wobei der mind. eine (erste) Hauptreaktor (200, 201)
- einen ersten Strömungsweg für das Stoffgemisch
über die immobile Katalysatorpackung und
- einen weiteren hiervon getrennten, geschlossenen Strömungsweg für ein Wärmetauschmedium außerhalb der Katalysatorpackung umfasst, und
wobei in den Medienumlauf ein Wärmetauscher (202) eingebunden ist;
- die Trenneinheit (106) mind.
- eine erste Trennstufe (106A) zur (im Wesentlichen) Abtrennung des Lösungsmittels
und
- eine zweite Trennstufe (106B) zur Abtrennung vom mind. einem Edukt und/oder mind. einem Nebenprodukt vom Produkt umfasst,
**dadurch gekennzeichnet, dass**
i) ein Sammelkreislauf (550) für einen geschlossenen ersten Medienumlauf umfasst ist, in den als mind. eine Wärmequelle der Wärmetauscher (202) des Hauptreaktors (200) und/oder mind. ein Wärmetauscher (354) der Trenneinheit (106) sowie ein erster Verdampfer (170) eingebunden sind,
ii) ein Zwischenkreislauf (560) für einen geschlossenen zweiten Medienumlauf umfasst ist, in den der erste Verdampfer (170), mind. ein Verdichter (173) und ein zweiter Verdampfer (174) eingebunden sind, und wobei
iii) mind. ein Verteilerkreislauf (570) für einen geschlossenen dritten Medienumlauf umfasst ist, in den als Wärmesenke mind. ein Wärmetauscher (206) der Reaktoreinheit (102), mind. ein Wärmetauscher (158) der der Konditionierungseinheit (104) und/oder mind. ein Wärmetauscher (302) der Trenneinheit (106) wärmetauschen eingebunden ist.

2. Anlage nach Anspruch 1, **dadurch gekennzeichnet, dass** im Zwischenkreislauf (560) ein Wärmetauscher (175) kreuzweise in den hinführenden Leitungsast (561) und den rückführenden Leitungsast (562) eingebunden ist, wobei der erste Leitungsast (561) einen ersten Innenraum des Wärmetauschers (175) und der rückführende Leitungsast (562) in den zweiten Innenraum des Wärmetauschers (175) eingebunden ist.

3. Anlage nach Anspruch 1 oder 2, **dadurch gekennzeichnet, dass** im hinführenden Leitungsast (561) des Zwischenkreislaufs (560) mind. zwei Verdichter (173, 174) eingebunden sind.

4. Anlage nach einem der vorherigen Ansprüche, **dadurch gekennzeichnet, dass** im Zwischenkreislauf (560) mind. eine querführende Leitung (563) zwischen dem hinführenden und rückführenden Leitungsast (561, 562) vorgesehen ist, die aus dem rückführenden Leitungsast (562) an einem Leitungsknoten abzweigt und an einem Leitungsknoten in den hinführenden Leitungsast (561) einleitet, wobei die Abzweigung vorteilhafterweise zwischen dem Wärmetauscher (175) und dem ersten Verdampfer (170) angeordnet ist, und die Einleitung entweder zwischen dem Wärmetauscher (175) und dem mind. einen Verdichter (137) oder zwischen zwei Verdichtern (173, 174) in den hinführenden Leitungsast (561) eingebunden ist.

5. Anlage nach einem der vorherigen Ansprüche, **dadurch gekennzeichnet, dass** in den Medienumlauf des Sammelkreislaufs (550) ein Sammeltank (180) und eine Pumpe (182) eingebunden sind, wobei ein Wärmetauscher (182) im rückführenden Leitungsast (552) stromaufwärts zum Sammeltank (180) und/oder im hinführenden Leitungsast (551) auf der Saugseite der Pumpe (182) angeordnet ist.

6. Anlage nach einem der vorherigen Ansprüche, **dadurch gekennzeichnet, dass** der Verteilerkreislaufs (570) einen ersten Leitungsast (571) als dampfführende Zuleitung umfasst, wobei in den zuführenden Leitungsast (571) mind. ein Verdichter (192, 193, 194) eingebunden ist.

7. Anlage nach Anspruch 6, **dadurch gekennzeichnet, dass** vom zweiten Verdampfer (172) eine (Sumpf-)Leitung (574) auf die Druckseite des eines Verdichters (192) und auf die Saugseite eines stromabwärts befindlichen weiteren Verdichters (193) führt, wobei in diese (Sumpf-)Leitung (574) eine Pumpe (191) eingebunden ist und/oder die (Sumpf-)Leitung (574) oder mind. eine Ast der (Sumpf-)Leitung (574) vom zweiten Verdampfer (172) in den hinführenden Leitungsast (571) zwischen zwei Verdichtern (192, 193, 194) geführt ist, insb. die (Sumpf-)Leitung (574) mind. zwei Äste aufweist, wobei jeder Ast der (Sumpf-)Leitung (574) zwischen zwei der Verdichter (192, 193, 194) geführt ist, und wobei mind. ein Ast der (Sumpf-)Leitung (574) einen Druckregler (196, 197) umfasst.

8. Anlage nach einem der vorherigen Ansprüche, **dadurch gekennzeichnet, dass** der Verteilerkreislaufs (570) einen weiteren Leitungsast (572) zur Verteilung- oder Abgabe von Energie und einen weiteren Leitungsast (573) zur Medienrückführung umfasst, wobei in den Leitungsast (572) zur Verteilung mind. ein Wärmetauscher (206) der Reaktoreinheit (102), mind. eine Wärmetauscher (158) der Konditionierungseinheit (104) und/oder mind. ein Wärmetauscher (302) der Trenneinheit (106) als Wärmesenke eingebunden ist, insb. eine Mehrzahl von den jeweiligen Wärmetauschern eingebunden sind.

9. Anlage nach einem der vorherigen Ansprüche, **dadurch gekennzeichnet, dass** in den rückführenden Leitungsast (573) des Verteilerkreislaufs (570) ein Wärmetauscher (199) wärmetauschend eingebunden ist.

10. Anlage nach einem der vorherigen Ansprüche, **dadurch gekennzeichnet, dass** mind.
eine Teilzahl der als Wärmesenke eingebundenen Wärmetauscher (158, 206, 302) des Verteilerkreislaufs (570) parallelgeschaltet sind, insb. jeweils im Zu- und/oder Ablauf des Mediums eingebunden und steuer- und/oder regelbar sind.

11. Anlage nach einem der vorherigen Ansprüche, **dadurch gekennzeichnet, dass** die als Wärmequelle wirkenden Wärmetauscher (202, 354) des Sammelkreislaufs (550) in Reihe geschaltet sind.

12. Verfahren zur katalytischen Hydrierung von Methylendianilin (MDA; Edukt1) mit einem Wasserstoffgeber (Edukt2), insb. einem gasförmigen Wasserstoffgeber, vorzugsweises Wasserstoff (H2),
wobei die Herstellung mittels einer industriellen Anlage erfolgt,
**dadurch gekennzeichnet, dass**
die Anlage nach mindestens einem der vorstehenden Vorrichtungsansprüche ausgebildet ist, wobei der Hauptreaktor (200) bei einer Temperatur im Bereich von 80 °C bis 150 °C betrieben wird, und wobei im Zwischenkreislauf (560) mittels des mind. einen Verdichters (173)) durch Dampfkompression mind. eine Temperaturerhöhung des Mediums in dem hinführenden Leitungsast (561) von 30 °C bis 120 °C vorgenommen wird, idealerweise von 50 °C bis 90 °C.

13. Verfahren nach Anspruch 12, **dadurch gekennzeichnet, dass** die Temperatur des Eduktstroms am Einlass des Hauptreaktors (200, 201) 90 bis 140°C beträgt, idealerweise 100 bis 135 °C.

14. Verfahren nach Anspruch 12 oder 13, **dadurch gekennzeichnet, dass** der mind. eine Hauptreaktor (200) bei einem Druck im Bereich von 60 bar bis 120 bar betrieben wird, idealerweise im Bereich von 70 bis 110 bar.

15. Verfahren nach einem der vorherigen Verfahrensansprüche, **dadurch gekennzeichnet, dass** der Druck im Hauptreaktor (200, 201) 60 bis 120 bar beträgt, idealerweise 70 bis 110 bar.

16. Verfahren nach einem der vorherigen Verfahrensansprüche, **dadurch gekennzeichnet, dass** dieses kontinuierlich, katalytisch zur Produktion von Methylenbis(cyclohexylamin) erfolgt, insb. zur Produktion von 4,4`-Diaminodicyclohexylmethan (PACM), bevorzugt 4,4`-Diaminodicyclohexylmethan (PACM) mit niedrigen Anteilen an trans/trans-Isomeren.

17. Verfahren nach einem der vorherigen Verfahrensansprüche, **dadurch gekennzeichnet, dass** vom Zeitpunkt t₀, dem Verfahrensstart nach Erneuerung oder Regeneration des Katalysators bis zum Zeitpunkt t₄, dem Ende des Verfahrens, bestimmt durch die Erneuerung oder Regeneration des Katalysators, die Betriebstemperatur des Hauptreaktors erhöht und Temperatur im Stoffstrom im Einlass (Feed) des Nachreaktors gleichgehalten oder gesenkt wird, wobei die Temperaturerhöhung oder -Absenkung linear und/oder schrittweise erfolgt.

18. Verfahren nach einem der vorherigen Verfahrensansprüche, **dadurch gekennzeichnet, dass** im Verteilerkreislauf (570) eine mehrstufige Druckerhöhung in dem hinführende Leitungsast (571) erfolgt, wobei nach dem zweiten Verdampfer (172) und vor dem ersten Verdichter (192) in dem ersten Leitungsast (571) ein Eingangsdruck von 1,5 bar bis 5 bar und eine Temperatur von 100°C bis 150°C vorliegt.

19. Verfahren nach einem der vorherigen Verfahrensansprüche, **dadurch gekennzeichnet, dass** im Verteilerkreislauf (570) im ersten Leitungsast (571) eine mehrstufige Druckerhöhung erfolgt, wobei nach dem letzten Verdichter (194) und vor dem ersten als Wärmesenke wirkenden Wärmetauscher (302, 362) ein Druck von 3 bar bis 30bar und eine Temperatur von 130°C bis 300°C vorliegt.

20. Verfahren nach einem der vorherigen Verfahrensansprüche, **dadurch gekennzeichnet, dass** die vom Sammelkreislauf (550) in den ersten Verdampfer (170) eingebrachte Energie mittels des Zwischenkreislaufs (560) und dem mind. einen eingebundenen Verdichter (173, 174) sowie dem kreuzweise eingebundenen Wärmetauscher (175)
- um mind. Faktor 1,1 bis 2,5 und/oder
- die Ausgangstemperatur des ersten Verdampfers (170)
um mind. den Faktor 1,2 bis 3,0
angehoben wird.

21. Verfahren nach einem der vorherigen Verfahrensansprüche, **dadurch gekennzeichnet, dass** das MDA (Edukt1) eine Mischung der folgenden Monomere umfasst oder hieraus gebildet wird: 4,4'-MDA, 2,4'-MDA und 2,2'-MDA, wobei der Anteil an 4,4'-MDA vorteilhafterweise im Bereich von 75 bis 98 mol% beträgt, idealerweise 85 bis 95 mol%.
